(19) Europäisches Patentamt
European Patent Office
Office européen des brevets

(11) **EP 3 726 221 B1**

## (12) EUROPEAN PATENT SPECIFICATION

(45) Date of publication and mention
of the grant of the patent:
**02.08.2023 Bulletin 2023/31**

(21) Application number: **18851082.0**

(22) Date of filing: **30.08.2018**

(51) International Patent Classification (IPC):
**C12Q 1/6841** (2018.01)    **C12Q 1/6886** (2018.01)
**G01N 33/68** (2006.01)    **G01N 33/574** (2006.01)
**G01N 1/30** (2006.01)

(52) Cooperative Patent Classification (CPC):
**G01N 33/57419; C12Q 1/6886;** C12Q 2600/112;
C12Q 2600/158; G01N 1/30

(86) International application number:
**PCT/CN2018/103357**

(87) International publication number:
**WO 2019/042380 (07.03.2019 Gazette 2019/10)**

(54) **HIERARCHICAL MODEL FOR DETECTING BENIGN AND MALIGNANT DEGREES OF COLORECTAL TUMORS AND APPLICATION THEREOF**

HIERARCHISCHES MODELL ZUM NACHWEIS VON GUTARTIGEN UND BÖSARTIGEN KOLOREKTALEN TUMOREN UND DEREN VERWENDUNG

MODÈLE HIÉRARCHIQUE DE DÉTECTION DE DEGRÉS BÉNINS ET MALINS DE TUMEURS COLORECTALES ET APPLICATION DE CE DERNIER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.08.2017  CN 201710774223**
**16.08.2018  CN 201810934234**

(43) Date of publication of application:
**21.10.2020  Bulletin 2020/43**

(73) Proprietor: **Lisen Imprinting Diagnostics, Inc.**
**Dover, Delaware 19901 (US)**

(72) Inventors:
• **CHENG, Tong**
**Wuxi**
**Jiangsu 214000 (CN)**
• **ZHOU, Ning**
**Wuxi**
**Jiangsu 214000 (CN)**

(74) Representative: **Grund, Martin et al**
**Grund Intellectual Property Group**
**Patentanwälte und Solicitor PartG mbB**
**Steinsdorfstraße 2**
**80538 München (DE)**

(56) References cited:
**CN-A- 103 344 762    US-A1- 2013 209 446**

• **QING ZHONG ET AL: "Image-based computational quantification and visualization of genetic alterations and tumour heterogeneity", SCIENTIFIC REPORTS, vol. 6, no. 1, 1 April 2016 (2016-04-01), page 24146, XP055667022, DOI: 10.1038/srep24146**
• **FREDRIK ENLUND ET AL: "Molecular analyses of the candidate tumor suppressor gene, PLAGL1, in benign and malignant salivary gland tumors", EUROPEAN JOURNAL OF ORAL SCIENCES, vol. 112, no. 6, 1 December 2004 (2004-12-01), pages 545-547, XP055771077, DK ISSN: 0909-8836, DOI: 10.1111/j.1600-0722.2004.00174.x**
• **LIN F ET AL: "Evaluation of the expression and role of IGF pathway biomarkers in human sarcomas", INTERNATIONAL JOURNAL OF IMMUNOPATHOLOGY AND PHARMACOLOGY, SAGE PUBLICATIONS LTD, GB, vol. 26, no. 1, 31 December 2012 (2012-12-31), pages 169-177, XP009525319, ISSN: 0394-6320, DOI: 10.1177/039463201302600116 [retrieved on 2013-01-01]**

- JAN AHLÉN ET AL: "Insulin-Like Growth Factor Type 1 Receptor Expression Correlates to Good Prognosis in Highly Malignant Soft Tissue Sarcoma", CLINICAL CANCER RESEARCH, vol. 11, no. 1, 1 January 2005 (2005-01-01), pages 206-216, XP055771090,
- MINGZHAO XING: "Minireview: Gene Methylation in Thyroid Tumorigenesis", ENDOCRINOLOGY, vol. 148, no. 3, 1 March 2007 (2007-03-01) , pages 948-953, XP055683798, US ISSN: 0013-7227, DOI: 10.1210/en.2006-0927
- NISHIHARA, S.: "Multipoint imprinting analysis in sporadic colorectal can- cers with and without microsatellite instability", INTERNATIONAL JOURNAL OF ONCOLOGY, 1 August 2000 (2000-08-01), XP055579749,
- SILVIERA, M.L.: "Epigenetic Differences in Normal Colon Mucosa of Cancer Patients Suggest Altered Dietary Metabolic Pathways", Cancer Prev. Res., vol. 5, no. 3, 1 March 2012 (2012-03-01), pages 374-384, XP055581036,
- CALON, A.: "Stromal gene expression defines poor-prognosis subtypes in co- lorectal cancer", Nature Genetics, vol. 47, no. 4, 23 February 2015 (2015-02-23), pages 320-329, XP055297456, DOI: doi:10.1038/ng.3225
- IINO, H.: "DNA microsatellite instability and mismatch repair protein loss in adenomas presenting in hereditary non-polyposis colorectal cancer", Gut, vol. 47, no. 1, 1 July 2000 (2000-07-01), page 37, XP055581043,
- KOWALCZYK, A.E. et al.: "Altered expression of the PLAGL1 (ZAC1/LOT1) gene in co- lorectal cancer: Correlations to the clinicopathological parameters", INTERNATIONAL JOURNAL OF ONCOLOGY, vol. 47, no. 3, 30 September 2015 (2015-09-30), pages 951-962, XP055578711,
- ORTEGA P. et al: "MMP-7 and SGCE as distinctive molecular factors in sporadic colorectal cancers from the mutator phenotype pathway", INTERNATIONAL JOURNAL OF ONCOLOGY, vol. 36, no. 5, 1 May 2010 (2010-05-01), pages 1209-1215, XP055555564,
- BREG, J. S.: "Imprinted Genes That Regulate Early Mammalian Growth Are Coe- xpressed in Somatic Stem Cells", PLoS ONE, vol. 6, no. 10, 19 October 2011 (2011-10-19), page e26410, XP055581051,

**Description**

**FIELD**

The present disclosure relates to the field of biotechnology, such as the field of genetic diagnosis, and relates to for example a grading model for such as determining the benignity or malignancy of a colorectal tumor and uses thereof, such as a grading model for determining the benignity or malignancy of a colorectal tumor using a set of imprinted genes and a device therefor.

**BACKGROUND**

Colorectal cancer is one of the gastrointestinal malignancies with the highest incidence in the world. According to World Health Organization statistics, in 2012 there were 1.361 million new cases and 694,000 deaths of colorectal cancer worldwide, of which there were about 376,000 newly diagnosed cases and 191,000 deaths in China. Studies have found that more than 90% of colorectal cancers develop from intestinal polyp, and the incidence of intestinal polyp in people aged over 50 years in China is about 10%. Therefore, experts recommend that people aged over 50 years should undergo regular enteroscopy. The progression from intestinal polyp to invasive colorectal cancer lasts about 5-20 years. Early non-invasive colorectal cancer can be removed under enteroscopy, with a 5-year survival rate more than 80%. However, the 5-year survival rate of advanced colorectal cancer with systemic metastasis is less than 15%. Therefore, early diagnosis and early treatment of colorectal cancer is the key to saving patient with colorectal cancer. However, patients with early colorectal cancer usually do not show specific symptoms, and even 30% of patients have no symptoms at all. In addition, the commonly used stool occult blood tests have low sensitivity and specificity. Therefore, a large proportion of patients with colorectal cancer are already in moderate or advanced stage when diagnosed. In addition, since all the current surgeries for colorectal cancer use a uniform standard to remove adjacent tissues thereof, it is impossible to accurately determine whether the malignant lesions have been completely removed or not, causing 30-40% patients undergoing surgery to relapse within 5 years. Therefore, the development of a more accurate detection technology is of great significance for the early diagnosis and treatment of colorectal cancer and for reducing the recurrence rate after surgery.

Traditional pathological diagnosis for benign and malignant cells is based on cell size, morphology, invasiveness and their relationship to surrounding cellular tissues, and however is greatly limited in the detection for early change in cancer cells. As a result, the cancer diagnosis method for cells at molecular level has once become a research hotspot. With the deeper continuous research in the field of molecular biology, more and more molecular detection technologies have been applied to cancer diagnosis.

Cancer is caused by uncontrolled cell growth/division resulting from epigenetic changes and genetic mutations that accumulate over time. Traditional pathological diagnosis for the benignity or malignancy of colorectal tumors is based on the variation in size, morphology, and structure of cell and tissue. With the development and deepening of molecular biology, more and more molecular detection technologies have been applied to the detection of colorectal cancer. From the analysis of the development of cancer, the changes at the molecular level (epigenetics and genetics) develop much earlier than the changes in cell morphology and tissue structure. Therefore, molecular biological tests are more sensitive in the early detection of cancer.

Based on the above reasons, in the current diagnosis of colorectal cancer, there is a need for a new detection system and model which analyze the changes in the molecular markers present in colorectal cancer cells in a biopsy sample of a patient, so as to provide more accurate pre-diagnosis and diagnostic information.

Zhong et al., Scientific Reports, 2016, 6(1) discloses a device suitable for determining benignity or malignancy of a tumor which comprises at least a detection unit for performing ISH as well as an analysis unit suitable for analyzing microscopic images and determining the expression level of imprinted genes.

**SUMMARY**

The invention is defined by the appended claims.

In order to achieve the above purpose, this application uses the following technical solutions:

The present disclosure provides a model for grading an imprinted gene in a colorectal tumor, comprising calculating the changes of total expression of the imprinted gene, of the expression of the imprinted gene with loss of imprinting, and of the expression of the imprinted gene with copy number variation in a colorectal cancer, to grade the expression of the imprinted gene;

Z1, Z4, Z5, Z6, Z8, Z10, Z11, Z13 or Z16, and wherein the imprinted gene Z1 is Gnas, the imprinted gene Z5 is Mest, the imprinted gene Z10 is Gatm, the imprinted gene Z11 is GrbIO, the imprinted gene Z4 is Igf2r, the imprinted

gene Z6 is PlagII, the imprinted gene Z8 is Dcn, the imprinted gene Z13 is Sgce, and the imprinted gene Z16 is Snrpn/Snurf.

Loss of imprinting refers to the activation (demethylation) of the allele in the imprinted gene that is previously silent, which is the most common and earliest epigenetic change in cancer, and this characteristic can be used as a pathological marker. In contrast, in healthy cells, the occurrence of imprinted gene with loss of imprinting is very low.

The inventors found that by calculating the expression of an imprinted gene with loss of imprinting and the expression of an imprinted gene with copy number variation for any one of imprinted genes Z1, Z5, Z10 and Z11 in a colorectal tumor, the diagnostic sensitivity to colorectal cancer can reach 84.6% or more.

In an embodiment which is not part of the invention, if only one imprinted gene is detected in the preliminary detection, it may be any one of the imprinted genes Z1, Z5, Z10, and Z11.

In an embodiment which is not part of the invention, if only one imprinted gene is detected in the preliminary detection, it may be any one of Z1 or Z11, further preferably Z11.

The inventors found: that if the single Z1 imprinted gene is detected, the diagnostic sensitivity to colorectal cancer can reach 92.1%; if the single Z5 imprinted gene is detected, the diagnostic sensitivity to colorectal cancer can reach 87.5%; if the single Z10 imprinted gene is detected, the diagnostic sensitivity to colorectal cancer can reach 84.6%; and if the single Z11 imprinted gene is detected, the diagnostic sensitivity to colorectal cancer can reach 92.9%.

In an embodiment which is not part of the invention, the calculation of the imprinted gene expression in the model comprises detecting any two of Z1, Z5, Z10 and Z11, and preferably a combination of Z1 and Z11, or a combination of Z1 and Z5, if a combination of two imprinted genes is detected.

The inventors found that by calculating the total expression of an imprinted gene, the expression of an imprinted gene with loss of imprinting and the expression of an imprinted gene with copy number variation for two or more of imprinted genes, the sensitivity can be further improved. When a combination of any two of imprinted genes Z1, Z5, Z10 and Z11 is detected, the diagnostic sensitivity to colorectal cancer can reach 95.8% or more; when a combination of Z1 and Z10 or a combination of Z5 and Z10 is detected, the diagnostic sensitivity to colorectal cancer can reach 95.8%; and when a combination of Z1 and Z11 or a combination of Z1 and Z5 is detected, the diagnostic sensitivity to colorectal cancer can reach 99.0% or more.

In an embodiment of the present disclosure, the imprinted gene further comprises any one or more of Z4, Z6, Z8, Z13 or Z16; wherein the imprinted gene Z4 is Igf2r, the imprinted gene Z6 is PlagI1, the imprinted gene Z8 is Dcn, the imprinted gene Z13 is Sgce, and the imprinted gene Z16 is Snrpn/Snurf.

The inventors found that by detecting Z4, Z6, Z8, Z13 and Z16 genes in addition to Z1, Z5, Z10 and Z11 genes to perform combination diagnosis, the accuracy of detection is increased, In addition, the addition of other probes can further avoid false positives in the diagnosis, further improving the detection accuracy, and thereby enabling accurate grading and diagnosis of all colorectal tumor samples.

In an embodiment of the present disclosure, the calculation of the imprinted gene expression in the model comprises calculating the expression of a combination of imprinted genes, wherein the combination is a combination of nine imprinted genes Z1, Z4, Z5, Z6, Z8, Z10, Z11, Z13 and Z16.

For an imprinted gene with loss of imprinting, after performing hematoxylin staining on a cell, there are two red/brown marks in the nucleus of the cell. For an imprinted gene with copy number variation, after performing hematoxylin staining on a cell, there are more than two red/brown markers in the nucleus of the cell. The copy number variation is caused by abnormal gene duplication in cancer cells, resulting in the expression of this gene as a triploid or even higher polyploid.

In an embodiment of the present disclosure, the expression of an imprinted gene, the expression of an imprinted gene with loss of imprinting and the expression of an imprinted gene with copy number variation are calculated by the following formulas:

$$\text{Total expression of an imprinted gene} = (b+c+d) \, / \, (a+b+c+d) \times 100\%;$$

$$\text{Expression of a normal imprinted gene} = b \, / \, (b+c+d) \times 100\%;$$

$$\text{Expression of an imprinted gene with loss of imprinting (LOI)} = c \, / \, (b+c+d) \times 100\%;$$

$$\text{Expression of an imprinted gene with copy number variation (CNV)} = d \, / \, (b+c+d) \times 100\%;$$

wherein,

a represents that after performing hematoxylin staining on a cell, there is no mark in the nucleus of the cell, the imprinted gene has no expression; b represents that after performing hematoxylin staining on a cell, there is one red/brown mark in the nucleus of the cell, the imprinted gene exists; c represents that after performing hematoxylin staining on a cell, there are two red/brown marks in the nucleus of the cell, the imprinted gene loses imprinting; and d represents that after performing hematoxylin staining on a cell, there are more than two red/brown markers in the nucleus of the cell, the imprinted gene has an copy number variation.

In an embodiment of the present disclosure, the mark after hematoxylin staining is, but not limited to, red or brown, and the marks after other staining method with other colors can also be used for calculating the total expression of an imprinted gene, the expression of an imprinted gene with loss of imprinting and the expression of an imprinted gene with copy number variation.

In an embodiment of the present disclosure, by *in situ* hybridization using a probe and hemotoxylin staining of the nucleus, the signal is amplified. Under a $40\times$ or $60\times$ microscope, the presence of imprinted gene, imprinted gene with loss of imprinting, or imprinted gene with copy number variation in each nucleus is determined. By calculating the expression of an imprinted gene with loss of imprinting and the expression of an imprinted gene with copy number variation, the benignity or malignancy of a tumor is determined. Due to the thickness of the section is only 10 $\mu$m, about 20% of the nuclei seen under the microscope are not intact, that is, there is a possibility of false negative results.

In an embodiment of the present disclosure, the total expression of an imprinted gene, the expression of an imprinted gene with loss of imprinting and the expression of an imprinted gene with copy number variation are classified into 5 grades according to the total expression of an imprinted gene, the expression of an imprinted gene with loss of imprinting and the expression of an imprinted gene with copy number variation for nine imprinted genes Z1, Z4, Z5, Z6, Z8, Z10, Z11, Z13 and Z16, and the expression is based on the counting of at least 1,200 cells in the area with clear marks on a sample for each probe.

In an embodiment of the present disclosure, the expression of an imprinted gene with loss of imprinting, the expression of an imprinted gene with copy number variation, and the total expression of an imprinted gene for Z1, Z11 or Z16 are classified into 5 grades:

Grade 0: any one or more of: the expression of Z1, Z11 or Z16 with loss of imprinting of less than 15%, the expression of Z1, Z11 or Z16 with copy number variation of less than 0.8%, or the total expression of Z1, Z11 or Z16 of less than 20%;

Grade I: any one or more of: the expression of Z1, Z11 or Z16 with loss of imprinting of 15-20%, the expression of Z1, Z11 or Z16 with copy number variation of 0.8-2%, or the total expression of Z1, Z11 or Z16 of 20-30%;

Grade II: any one or more of: the expression of Z1, Z11 or Z16 with loss of imprinting of 20-25%, the expression of Z1, Z11 or Z16 with copy number variation of 2-3.5%, or the total expression of Z1, Z11 or Z16 of 30-40%;

Grade III: any one or more of: the expression of Z1, Z11 or Z16 with loss of imprinting of 25-30%, the expression of Z1, Z11 or Z16 with copy number variation of 3.5-5%, or the total expression of Z1, Z11 or Z16 of 40-50%; and

Grade IV: any one or more of: the expression of Z1, Z11 or Z16 with loss of imprinting of more than 30%, the expression of Z1, Z11 or Z16 with copy number variation of more than 5%, or the total expression of Z1, Z 11 or Z16 of more than 50%;

wherein the expression of an imprinted gene with loss of imprinting, the expression of an imprinted gene with copy number variation, and the total expression of an imprinted gene for Z1, Z11 or Z16 are independent of each other.

In an embodiment of the present disclosure, the expression of an imprinted gene with loss of imprinting, the expression of an imprinted gene with copy number variation, and the total expression of an imprinted gene for Z4, Z5, Z6, Z8, Z10 or Z13 are classified into 5 grades:

Grade 0: any one or more of: the expression of Z4, Z5, Z6, Z8, Z10 or Z13 with loss of imprinting of less than 10%, the expression of Z4, Z5, Z6, Z8, Z10 or Z13 with copy number variation of less than 0.8%, or the total expression of Z4, Z5, Z6, Z8, Z10 or Z13 of less than 15%;

Grade I: any one or more of: the expression of Z4, Z5, Z6, Z8, Z10 or Z13 with loss of imprinting of 10-20%, the expression of Z4, Z5, Z6, Z8, Z10 or Z13 with copy number variation of 0.8-1.5%, or the total expression of Z4, Z5, Z6, Z8, Z10 or Z13 of 15-20%;

Grade II: any one or more of: the expression of Z4, Z5, Z6, Z8, Z10 or Z13 with loss of imprinting of 20-25%, the expression of Z4, Z5, Z6, Z8, Z10 or Z13 with copy number variation of 1.5-3%, or the total expression of Z4, Z5, Z6, Z8, Z10 or Z13 of 20-30%;

Grade III: any one or more of: the expression of Z4, Z5, Z6, Z8, Z10 or Z13 with loss of imprinting of 25-30%, the expression of Z4, Z5, Z6, Z8, Z10 or Z13 with copy number variation of 3-5%, or the total expression of Z4, Z5, Z6, Z8, Z10 or Z13 of 30-40%; and

Grade IV: any one or more of: the expression of Z4, Z5, Z6, Z8, Z10 or Z13 with loss of imprinting of more than 30%, the expression of Z4, Z5, Z6, Z8, Z10 or Z13 with copy number variation of more than 5%, or the total expression of Z4, Z5, Z6, Z8, Z10 or Z13 of more than 40%;

wherein the expression of an imprinted gene with loss of imprinting, the expression of an imprinted gene with copy number variation, and the total expression of an imprinted gene for Z4, Z5, Z6, Z8, Z10 or Z13 are independent of each other.

In an embodiment of the present disclosure, the present disclosure provides a device for determining the benignity or malignancy of a colorectal tumor by using the model, comprising the following units:

(1) sample unit for obtaining a test sample;

(2) probe design unit for designing a probe specific for the sequence of an imprinted gene;

(3) detection unit for performing *in situ* hybridization of the probe of step (2) to the test sample;

(4) analysis unit for analyzing microscopic images and determining the expression level of the imprinted gene; wherein, the analysis unit is operated by calculating the total expression of the imprinted gene, the expression of the imprinted gene with loss of imprinting, and the expression of the imprinted gene with copy number variation, using the model according to the first aspect, and grading the expression of the imprinted gene with loss of imprinting and the expression of the imprinted gene with copy number variation, to determine the benignity or malignancy of the colorectal tumor.

For an imprinted gene with loss of imprinting, after performing hematoxylin staining on a cell, there are two red/brown marks in the nucleus of the cell. For an imprinted gene with copy number variation, after performing hematoxylin staining on a cell, there are more than two red/brown markers in the nucleus of the cell. The copy number variation is caused by abnormal gene duplication in cancer cells, resulting in the expression of this gene as a triploid or even higher polyploid.

The mark after hematoxylin staining is, but not limited to, red or brown, and the marks after other staining method with other colors can also be used for calculating the total expression of an imprinted gene, the expression of an imprinted gene with loss of imprinting and the expression of an imprinted gene with copy number variation.

The device is used to visually detect the changes of imprinted genes in colorectal tumors at cell and tissue levels in early stage, so as to determine benignity or malignancy thereof, which provides the most favorable treatment opportunities for patients with early colorectal tumors.

In an embodiment of the present disclosure, the present disclosure provides a method for determining the benignity or malignancy of a colorectal tumor by using the model or the device, comprising the steps of:

(1) obtaining a test sample;

(2) designing a probe specific for the sequence of an imprinted gene;

(3) performing *in situ* hybridization of the probe of step (2) to the test sample; and

(4) analyzing microscopic images and determining the expression level of the imprinted gene, so as to determine the benignity or malignancy of the colorectal tumor;

wherein, the analysis unit is operated by calculating the expression of the imprinted gene with loss of imprinting, the expression of the imprinted gene with copy number variation and the total expression of the imprinted gene, using the model, and grading the expression of the gene with loss of imprinting and the expression of the imprinted gene with copy number variation, to determine the benignity or malignancy of the colorectal tumor.

In an embodiment of the present disclosure, the test sample of step (1) is derived from human tissue and/or cells.

The test samples are feasible as long as the RNA is subjected to fixation in time. Those skilled in the art can select the treatment methods according to needs, which is not particularly limited here. The test samples described in the present disclosure include any one or more of issue paraffin sections, enteroscopy biopsy samples and/or exfoliated cells in stool.

The specific operation steps of the paraffin section of the tissue are as follows: obtaining a human tumor tissue sample, fixing the sample with 10% neutral buffered formalin in time, embedding the sample into paraffin, cutting into a section with a thickness of 10 $\mu$m, and loading onto a positively charged slide as a tissue section. Because the section is only 10 $\mu$m thick, some of the nuclei are not intact under the microscope, and so some false negative results maybe occur for imprinted gene with loss of imprinting.

The specific operation steps for obtaining the enteroscopy biopsy sample are: obtaining human cells under an endoscope, and fixing them with 10% neutral buffered formalin in time.

The specific operation steps for obtaining exfoliated cells in stool are: obtaining intestinally exfoliated cells in stool, and fixing them with 10% neutral buffered formalin in time.

Because enteroscopy biopsy is less invasive to patients, is simple in the sampling process, and further can be site-directed as compared with blood with the circulation characteristics, enteroscopy biopsy has special advantages as a test sample.

Because exfoliated cells in stool are not invasive to the patients and are easily sampled, exfoliated cells in stool have special advantages as a test sample.

In an embodiment of the present disclosure, the test sample is an enteroscopy biopsy sample and/or exfoliated cells in stool.

The imprinted genes include Z1, Z4, Z5, Z6, Z8, Z10, Z11, Z13 and Z16, wherein the imprinted gene Z1 is Gnas, the imprinted gene Z4 is Igf2r, the imprinted gene Z5 is Mest, the imprinted gene Z6 is Plagl1, the imprinted gene Z8 is Dcn, the imprinted gene Z10 is Gatm, the imprinted gene Z11 is Grb10, the imprinted gene Z13 is Sgce, and the imprinted gene Z16 is Snrpn/Snurf.

The imprinted genes Z1(Gnas), Z4(Igf2r), Z5(Mest), Z6(Plagl1), Z8(Dcn), Z10(Gatm), Z11(Grb10), Z13(Sgce) and Z16(Snrpn/Snurf) are expressed to varying degrees in tumor tissues, and when a malignant lesion occurs, the expression level and imprinting status will change significantly.

The probes are designed according to the imprinted genes Z1, Z4, Z5, Z6, Z8, Z10, Z11, Z13 and Z16, namely Gnas, Igf2r, Mest, Plagl1, Dcn, Gatm, Grb10, Sgce and Snrpn/Snurf. Specifically, a sequence within an intron of each gene is selected as a probe. The specific probes are designed by Advanced Cell Diagnostics.

In an embodiment of the present disclosure, the *in situ* hybridization is RNAscope *in situ* hybridization.

In an embodiment of the present disclosure, the RNAscope *in situ* hybridization is performed by using singleplex or multiplex color assay kit or singleplex or multiplex fluorescence assay kit, preferably singleplex red/brown color assay kit or multiplex fluorescence assay kit.

The multiplex color assay kit or multiplex fluorescence assay kit include two or more channels of color assay kit or fluorescence assay kit. The two channel color assay kit or multiple channel fluorescence assay kit can use two imprinted gene probes to detect the expression of an imprinted gene and another gene or even the expression of multiple imprinted genes and non-imprinted genes.

In an embodiment of the present disclosure, the expression of an imprinted gene, the expression of an imprinted gene with loss of imprinting and the expression of an imprinted gene with copy number variation in the model are calculated by the following formulas:

$$\text{Total expression of an imprinted gene} = (b+c+d) / (a+b+c+d) \times 100\%;$$

$$\text{Expression of a normal imprinted gene} = b / (b+c+d) \times 100\%;$$

Expression of an imprinted gene with loss of imprinting (LOI) = c / (b+c+d) × 100%;

Expression of an imprinted gene with copy number variation (CNV) = d / (b+c+d) × 100%;

wherein,

a represents that after performing hematoxylin staining on a cell, there is no mark in the nucleus of the cell, the imprinted gene has no expression; b represents that after performing hematoxylin staining on a cell, there is one red/brown mark in the nucleus of the cell, the imprinted gene exists; c represents that after performing hematoxylin staining on a cell, there are two red/brown marks in the nucleus of the cell, the imprinted gene loses imprinting; and d represents that after performing hematoxylin staining on a cell, there are more than two red/brown markers in the nucleus of the cell, the imprinted gene has an copy number variation.

The mark after hematoxylin staining is, but not limited to, red or brown, and the marks after other staining method with other colors can also be used for calculating the total expression of an imprinted gene, the expression of an imprinted gene with loss of imprinting and the expression of an imprinted gene with copy number variation.

By *in situ* hybridization using a probe and hemotoxylin staining of the nucleus, the signal is amplified. Under a 40× or 60× microscope, the presence of imprinted gene, imprinted gene with loss of imprinting, or imprinted gene with copy number variation in each nucleus is determined. By calculating the total expression of an imprinted gene, the expression of an imprinted gene with loss of imprinting and the expression of an imprinted gene with copy number variation, the benignity or malignancy of a tumor is determined. Due to the thickness of the section is only 10 μm, about 20% of the nuclei seen under the microscope are not intact, that is, there is a possibility of false negative results.

In an embodiment of the present disclosure, the expression of an imprinted gene with loss of imprinting, the expression of an imprinted gene with copy number variation and the total expression of an imprinted gene are classified into 5 grades.

In an embodiment of the present disclosure, the 5 grades are classified according to the expression of an imprinted gene with loss of imprinting, the expression of an imprinted gene with copy number variation and the total expression of an imprinted gene for nine imprinted genes Z1, Z4, Z5, Z6, Z8, Z10, Z11, Z13 and Z16, and the expression is based on the counting of at least 1,200 cells in the area with clear marks on a sample for each probe.

The expression of an imprinted gene with loss of imprinting, the expression of an imprinted gene with copy number variation, and the total expression of an imprinted gene for Z1, Z11 or Z16 are classified into 5 grades:

Grade 0: any one or more of: the expression of Z1, Z11 or Z16 with loss of imprinting of less than 15%, the expression of Z1, Z11 or Z16 with copy number variation of less than 0.8%, or the total expression of Z1, Z11 or Z16 of less than 20%;

Grade I: any one or more of: the expression of Z1, Z11 or Z16 with loss of imprinting of 15-20%, the expression of Z1, Z11 or Z16 with copy number variation of 0.8-2%, or the total expression of Z1, Z11 or Z16 of 20-30%;

Grade II: any one or more of: the expression of Z1, Z11 or Z16 with loss of imprinting of 20-25%, the expression of Z1, Z11 or Z16 with copy number variation of 2-3.5%, or the total expression of Z1, Z11 or Z16 of 30-40%;

Grade III: any one or more of: the expression of Z1, Z11 or Z16 with loss of imprinting of 25-30%, the expression of Z1, Z11 or Z16 with copy number variation of 3.5-5%, or the total expression of Z1, Z11 or Z16 of 40-50%; and

Grade IV: any one or more of: the expression of Z1, Z11 or Z16 with loss of imprinting of more than 30%, the expression of Z1, Z11 or Z16 with copy number variation of more than 5%, or the total expression of Z1, Z11 or Z16 of more than 50%;

wherein the expression of an imprinted gene with loss of imprinting, the expression of an imprinted gene with copy number variation, and the total expression of an imprinted gene for Z1, Z11 or Z16 are independent of each other.

The expression of an imprinted gene with loss of imprinting, the expression of an imprinted gene with copy number variation, and the total expression of an imprinted gene for Z4, Z5, Z6, Z8, Z10 or Z13 are classified into 5 grades:

Grade 0: any one or more of: the expression of Z4, Z5, Z6, Z8, Z10 or Z13 with loss of imprinting of less than 10%, the expression of Z4, Z5, Z6, Z8, Z10 or Z13 with copy number variation of less than 0.8%, or the total expression of Z4, Z5, Z6, Z8, Z10 or Z13 of less than 15%;

Grade I: any one or more of: the expression of Z4, Z5, Z6, Z8, Z10 or Z13 with loss of imprinting of 10-20%, the expression of Z4, Z5, Z6, Z8, Z10 or Z13 with copy number variation of 0.8-1.5%, or the total expression of Z4, Z5, Z6, Z8, Z10 or Z13 of 15-20%;

Grade II: any one or more of: the expression of Z4, Z5, Z6, Z8, Z10 or Z13 with loss of imprinting of 20-25%, the expression of Z4, Z5, Z6, Z8, Z10 or Z13 with copy number variation of 1.5-3%, or the total expression of Z4, Z5, Z6, Z8, Z10 or Z13 of 20-30%;

Grade III: any one or more of: the expression of Z4, Z5, Z6, Z8, Z10 or Z13 with loss of imprinting of 25-30%, the expression of Z4, Z5, Z6, Z8, Z10 or Z13 with copy number variation of 3-5%, or the total expression of Z4, Z5, Z6, Z8, Z10 or Z13 of 30-40%; and

Grade IV: any one or more of: the expression of Z4, Z5, Z6, Z8, Z10 or Z13 with loss of imprinting of more than 30%, the expression of Z4, Z5, Z6, Z8, Z10 or Z13 with copy number variation of more than 5%, or the total expression of Z4, Z5, Z6, Z8, Z10 or Z13 of more than 40%;

wherein the expression of an imprinted gene with loss of imprinting, the expression of an imprinted gene with copy number variation, and the total expression of an imprinted gene for Z4, Z5, Z6, Z8, Z10 or Z13 are independent of each other.

In an embodiment of the present disclosure, the benignity or malignancy of the colorectal tumor is classified as benign tumor, potential colorectal cancer, early colorectal cancer, moderate colorectal cancer, or advanced colorectal cancer.

In an embodiment of the present disclosure, the colorectal tumor is determined as a benign tumor, if the expression of the imprinted genes Z1, Z4, Z5, Z6, Z8, Z10, Z11, Z13 and Z16 with loss of imprinting and the expression of the imprinted genes Z1, Z4, Z5, Z6, Z8, Z10, Z11, Z13 and Z16 with copy number variation both are less than Grade I, or if the expression of no more than one of the imprinted genes Z1, Z4, Z5, Z6, Z8, Z10, Z11, Z13 and Z16 with loss of imprinting is Grade I and the expression of no more than one of the imprinted genes Z1, Z4, Z5, Z6, Z8, Z10, Z11, Z13 and Z16 with copy number variation is Grade I;

the colorectal tumor is determined as a potential colorectal cancer, if the expression of at least two of the imprinted genes Z1, Z4, Z5, Z6, Z8, Z10, Z11, Z13 and Z16 with loss of imprinting is Grade I, if the expression of at least two of the imprinted genes Z1, Z4, Z5, Z6, Z8, Z10, Z11, Z13 and Z16 with copy number variation is Grade I, or if the expression of no more than one of the imprinted genes Z1, Z4, Z5, Z6, Z8, Z10, Z11, Z13 and Z16 with loss of imprinting is Grade II and the expression of no more than one of the imprinted genes Z1, Z4, Z5, Z6, Z8, Z10, Z11, Z13 and Z16 with copy number variation is Grade II;

the colorectal tumor is determined as an early colorectal cancer, if the expression of at least two of the imprinted genes Z1, Z4, Z5, Z6, Z8, Z10, Z11, Z13 and Z16 with loss of imprinting is Grade II, if the expression of at least two of the imprinted genes Z1, Z4, Z5, Z6, Z8, Z10, Z11, Z13 and Z16 with copy number variation is Grade II, or if the expression of no more than one of the imprinted genes Z1, Z4, Z5, Z6, Z8, Z10, Z11, Z13 and Z16 with loss of imprinting is Grade III and the expression of no more than one of the imprinted genes Z1, Z4, Z5, Z6, Z8, Z10, Z11, Z13 and Z16 with copy number variation is Grade III;

the colorectal tumor is determined as a moderate colorectal cancer, if the expression of at least two of the imprinted genes Z1, Z4, Z5, Z6, Z8, Z10, Z11, Z13 and Z16 with loss of imprinting is Grade III, if the expression of at least two of the imprinted genes Z1, Z4, Z5, Z6, Z8, Z10, Z11, Z13 and Z16 with copy number variation is Grade III, or if the expression of no more than one of the imprinted genes Z1, Z4, Z5, Z6, Z8, Z10, Z11, Z13 and Z16 with loss of imprinting is Grade IV and the expression of no more than one of the imprinted genes Z1, Z4, Z5, Z6, Z8, Z10, Z11, Z13 and Z16 with copy number variation is Grade IV; or

the colorectal tumor is determined as an advanced colorectal cancer, if the expression of at least two of the imprinted genes Z1, Z4, Z5, Z6, Z8, Z10, Z11, Z13 and Z16 with loss of imprinting is Grade IV, or if the expression of at least two of the imprinted genes Z1, Z4, Z5, Z6, Z8, Z10, Z11, Z13 and Z16 with copy number variation is Grade IV

In an embodiment of the present disclosure, the present disclosure provides use of the model or the device for determination of a colorectal tumor.

In an embodiment of the present disclosure, the present disclosure provides use of the model or the device for manufacture of a medicament or an apparatus for treating a colorectal tumor.

In an embodiment of the present disclosure, the benignity or malignancy of the colorectal tumor is classified as benign tumor, potential colorectal cancer, early colorectal cancer, moderate colorectal cancer, or advanced colorectal cancer.

In an embodiment of the present disclosure, the colorectal tumor is determined as a benign tumor, if the expression of the imprinted genes Z1, Z4, Z5, Z6, Z8, Z10, Z11, Z13 and Z16 with loss of imprinting and the expression of the imprinted genes Z1, Z4, Z5, Z6, Z8, Z10, Z11, Z13 and Z16 with copy number variation both are less than Grade I, or if the expression of no more than one of the imprinted genes Z1, Z4, Z5, Z6, Z8, Z10, Z11, Z13 and Z16 with loss of imprinting is Grade I and the expression of no more than one of the imprinted genes Z1, Z4, Z5, Z6, Z8, Z10, Z11, Z13 and Z16 with copy number variation is Grade I;

the colorectal tumor is determined as a potential colorectal cancer, if the expression of at least two of the imprinted genes Z1, Z4, Z5, Z6, Z8, Z10, Z11, Z13 and Z16 with loss of imprinting is Grade I, if the expression of at least two of the imprinted genes Z1, Z4, Z5, Z6, Z8, Z10, Z11, Z13 and Z16 with copy number variation is Grade I, or if the expression of no more than one of the imprinted genes Z1, Z4, Z5, Z6, Z8, Z10, Z11, Z13 and Z16 with loss of imprinting is Grade II and the expression of no more than one of the imprinted genes Z1, Z4, Z5, Z6, Z8, Z10, Z11, Z13 and Z16 with copy number variation is Grade II;

the colorectal tumor is determined as an early colorectal cancer, if the expression of at least two of the imprinted genes Z1, Z4, Z5, Z6, Z8, Z10, Z11, Z13 and Z16 with loss of imprinting is Grade II, if the expression of at least two of the imprinted genes Z1, Z4, Z5, Z6, Z8, Z10, Z11, Z13 and Z16 with copy number variation is Grade II, or if the expression of no more than one of the imprinted genes Z1, Z4, Z5, Z6, Z8, Z10, Z11, Z13 and Z16 with loss of imprinting is Grade III and the expression of no more than one of the imprinted genes Z1, Z4, Z5, Z6, Z8, Z10, Z11, Z13 and Z16 with copy number variation is Grade III;

the colorectal tumor is determined as a moderate colorectal cancer, if the expression of at least two of the imprinted genes Z1, Z4, Z5, Z6, Z8, Z10, Z11, Z13 and Z16 with loss of imprinting is Grade III, if the expression of at least two of the imprinted genes Z1, Z4, Z5, Z6, Z8, Z10, Z11, Z13 and Z16 with copy number variation is Grade III, or if the expression of no more than one of the imprinted genes Z1, Z4, Z5, Z6, Z8, Z10, Z11, Z13 and Z16 with loss of imprinting is Grade IV and the expression of no more than one of the imprinted genes Z1, Z4, Z5, Z6, Z8, Z10, Z11, Z13 and Z16 with copy number variation is Grade IV; or

the colorectal tumor is determined as an advanced colorectal cancer, if the expression of at least two of the imprinted genes Z1, Z4, Z5, Z6, Z8, Z10, Z11, Z13 and Z16 with loss of imprinting is Grade IV, or if the expression of at least two of the imprinted genes Z1, Z4, Z5, Z6, Z8, Z10, Z11, Z13 and Z16 with copy number variation is Grade IV.

As compared with prior art, in the examples of the present disclosure, the detection model and device are used to intuitively present the expression of an imprinted gene in a sample from a patient with bladder tumor. By *in situ* labeling for an imprinted gene, a change of the imprinted gene is detected objectively, intuitively, early and precisely, and a quantitative model can be provided, so as to make a great contribution to molecular pathological diagnosis.

After reading and understanding the accompanying drawings and detailed description, other aspects of the present disclosure will be understood.

## BRIEF DESCRIPTION OF DRAWINGS

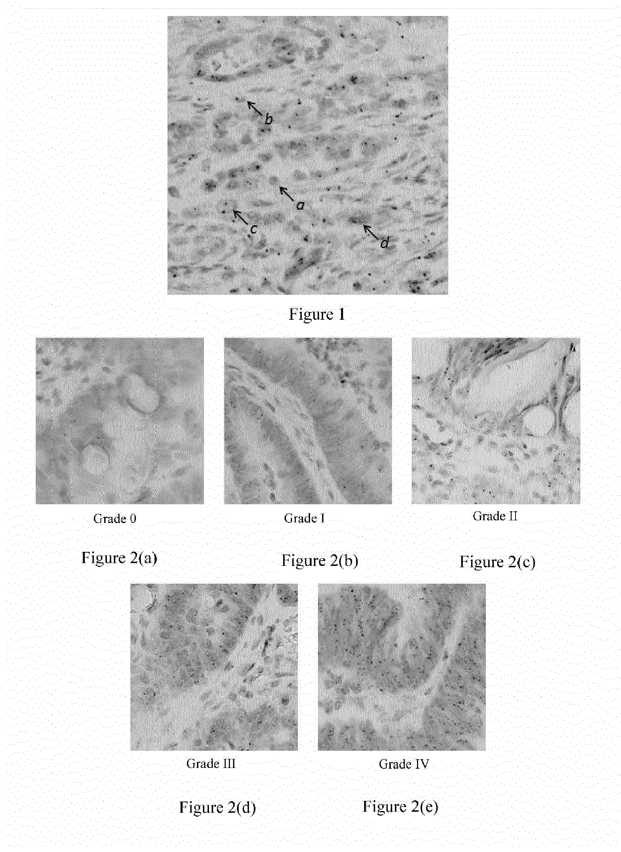

Figure 1 shows a pathological section of colorectal cancer stained with hematoxylin for nuclei, wherein, a represents that after performing hematoxylin staining on a cell, there is no mark in the nucleus of the cell, the imprinted gene has no expression; b represents that after performing hematoxylin staining on a cell, there is one red/brown mark in the nucleus of the cell, the imprinted gene exists; c represents that after performing hematoxylin staining on a cell, there are two red/brown marks in the nucleus of the cell, the imprinted gene loses imprinting; and d represents that after performing hematoxylin staining on a cell, there are more than two red/brown marks in the nucleus of the cell, the imprinted gene has copy number variation.

Figure 2(a) shows the expression of 9 genes in a pathological section of Grade 0 colorectal tumor; Figure 2(b) shows the expression of 9 genes in a pathological section of Grade I colorectal cancer; Figure 2(c) shows the expression of 9 genes in a pathological section of Grade II colorectal cancer; Figure 2(d) shows the expression of 9 genes in a pathological section of Grade III colorectal cancer; and Figure 2(e) shows the expression of 9 genes in a pathological section of Grade IV colorectal cancer.

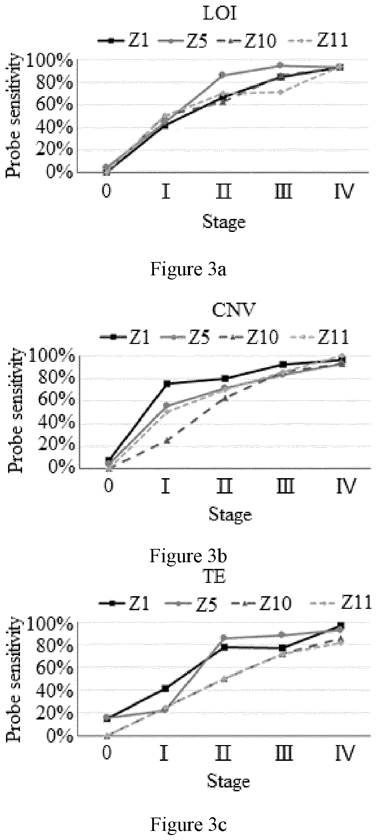

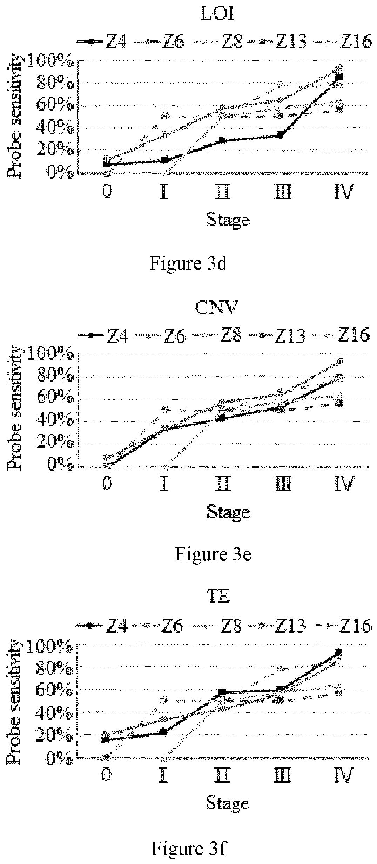

Figure 3(a) shows the expression of imprinted genes Z1, Z5, Z10 and Z11 with loss of imprinting in colorectal cancer; Figure 3(b) shows the expression of imprinted genes Z1, Z5, Z10 and Z11 with copy number variation in colorectal cancer; Figure 3(c) shows the total expression of imprinted genes Z1, Z5, Z10 and Z11 in colorectal cancer; Figure 3(d) shows the expression of imprinted genes Z4, Z6, Z8, Z13 and Z16 with loss of imprinting in colorectal cancer; Figure 3(e) shows the expression of imprinted genes Z4, Z6, Z8, Z13 and Z16 with copy number variation in colorectal cancer; and Figure 3(f) shows the total expression of imprinted genes Z4, Z6, Z8, Z13 and Z16 in colorectal cancer, wherein, LOI is the expression of an imprinted gene with loss of imprinting, CNV is the expression of an imprinted gene with copy number variation, and TE is the total expression of an imprinted gene.

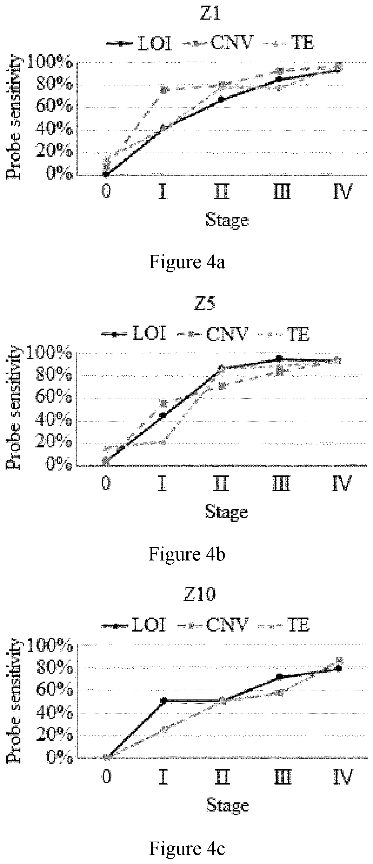

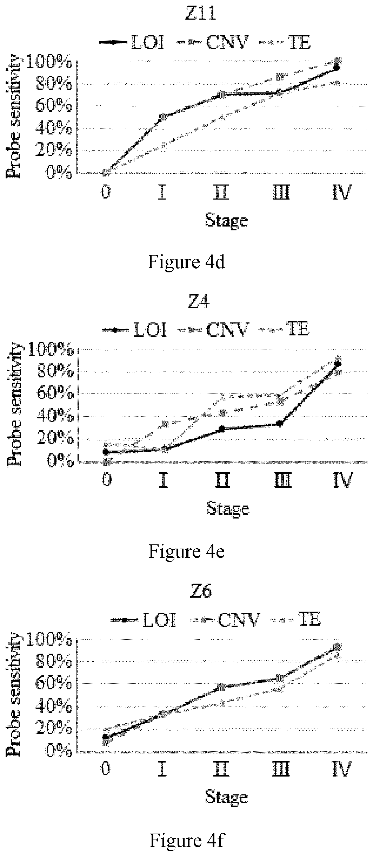

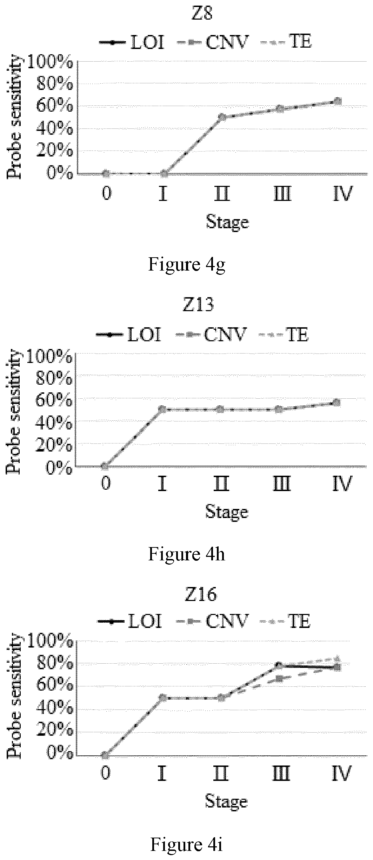

Figure 4(a) shows the expression of the imprinted gene Z1 with loss of imprinting, expression of the imprinted gene Z1 with copy number variation and total expression of the imprinted gene Z1; Figure 4(b) shows the expression of the imprinted gene Z5 with loss of imprinting, expression of the imprinted gene Z5 with copy number variation and total expression of the imprinted gene Z5; Figure 4(c) shows the expression of the imprinted gene Z10 with loss of imprinting, expression of the imprinted gene Z10 with copy number variation and total expression of the imprinted gene Z10; Figure 4(d) shows the expression of the imprinted gene Z11 with loss of imprinting, expression of the imprinted gene Z11 with copy number variation and total expression of the imprinted gene Z11; Figure 4(e) shows the expression of the imprinted gene Z4 with loss of imprinting, expression of the imprinted gene Z4 with copy number variation and total expression of the imprinted gene Z4; Figure 4(f) shows the expression of the imprinted gene Z6 with loss of imprinting, expression of the imprinted gene Z6 with copy number variation and total expression of the imprinted gene Z6; Figure 4(g) shows the expression of the imprinted gene Z8 with loss of imprinting, expression of the imprinted gene Z8 with copy number variation and total expression of the imprinted gene Z8; Figure 4(h) shows the expression of the imprinted gene Z13 with loss of imprinting, expression of the imprinted gene Z13 with copy number variation and total expression of the imprinted gene Z13; and Figure 4(i) shows the expression of the imprinted gene Z16 with loss of imprinting, expression of the imprinted gene Z16 with copy number variation and total expression of the imprinted gene Z16, wherein, LOI is the expression of an imprinted gene with loss of imprinting, CNV is the expression of an imprinted gene with copy number variation, and TE is the total expression of an imprinted gene.

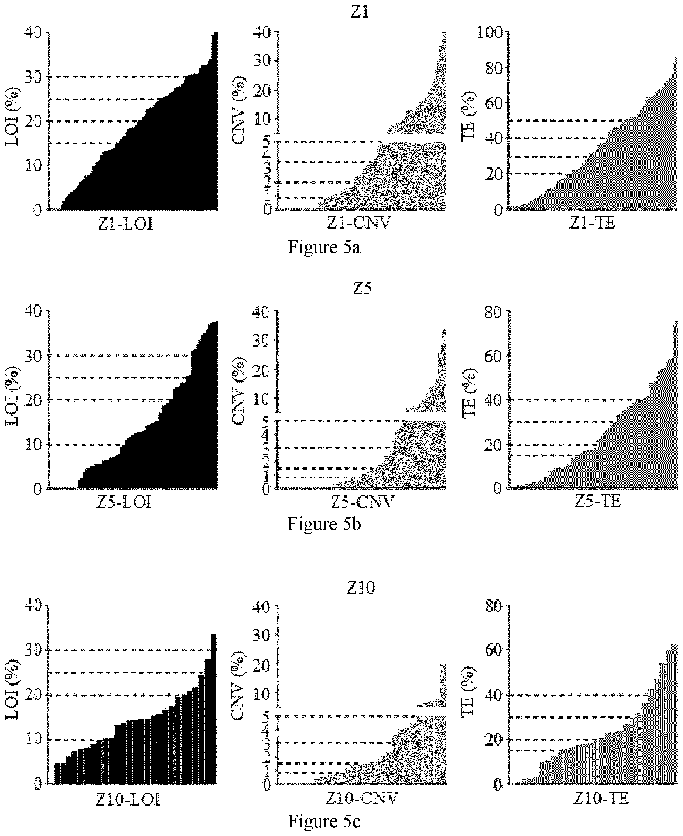

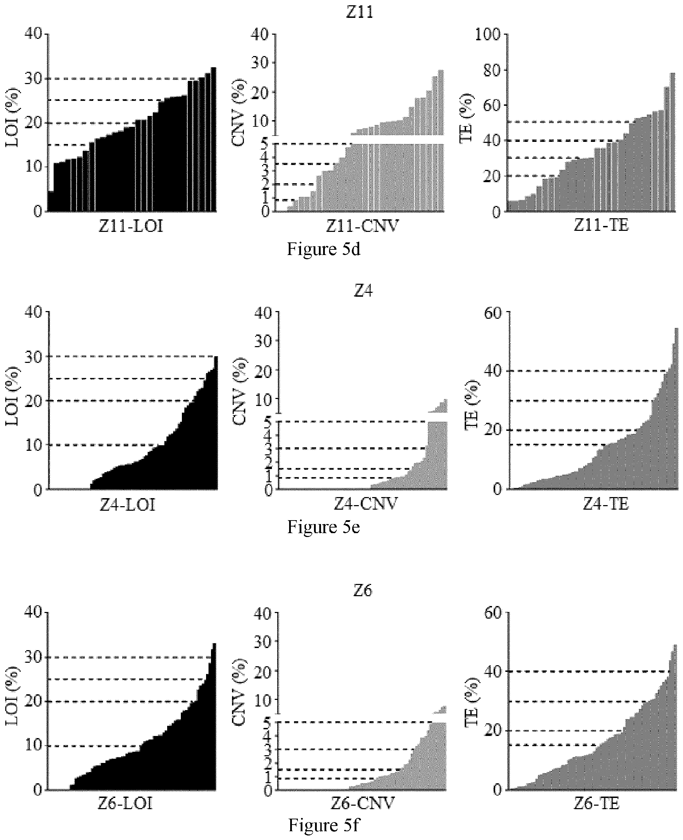

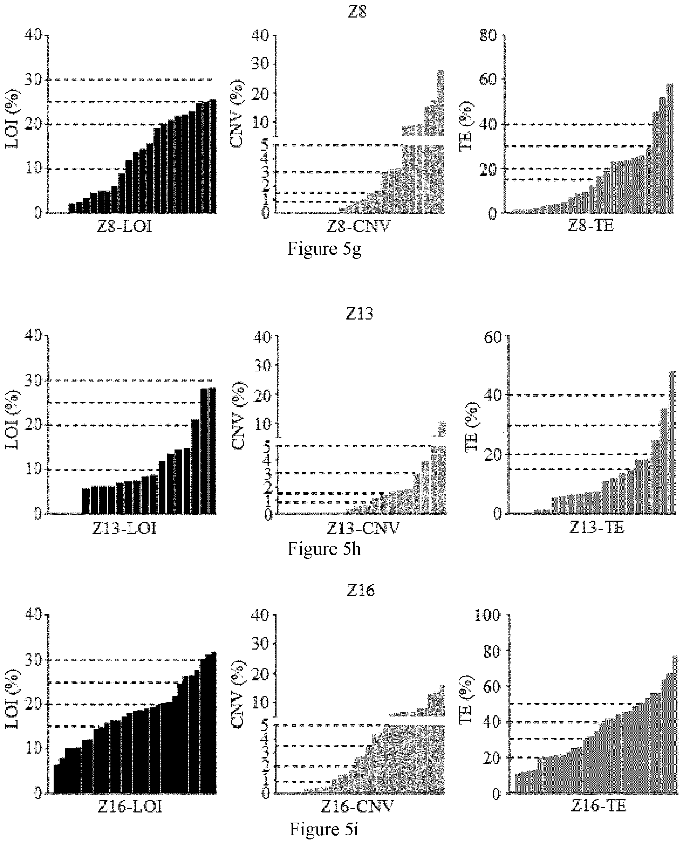

Figure 5(a) shows the distribution range and grading standard of Z1 with loss of imprinting and Z1 with copy number variation in pathological sections of 107 colorectal cancers; Figure 5(b) shows the distribution range and grading standard of Z5 with loss of imprinting and Z5 with copy number variation in pathological sections of 107 colorectal cancers; Figure 5(c) shows the distribution range and grading standard of Z10 with loss of imprinting and Z10 with copy number variation in pathological sections of 107 colorectal cancers; Figure 5(d) shows the distribution range and grading standard of Z11 with loss of imprinting and Z11 with copy number variation in pathological sections of 107 colorectal cancers; Figure 5(e) shows the distribution range and grading standard of Z4 with loss of imprinting and Z4 with copy number variation in pathological sections of 107 colorectal cancers; Figure 5(f) shows the distribution range and grading standard of Z6 with loss of imprinting and Z6 with copy number variation in pathological sections of 107 colorectal cancers; Figure 5(g) shows the distribution range and grading standard of Z8 with loss of imprinting and Z8 with copy number variation in pathological sections of 107 colorectal cancers; Figure 5(h) shows the distribution range and grading standard of Z13 with loss of imprinting and Z13 with copy number variation in pathological sections of 107 colorectal cancers; and Figure 5(i) shows the distribution range and grading standard of Z16 with loss of imprinting and Z16 with copy number variation in pathological sections of 107 colorectal cancers, wherein, LOI is the expression of an imprinted gene with loss of imprinting, CNV is the expression of an imprinted gene with copy number variation, and TE is the total expression of an imprinted gene.

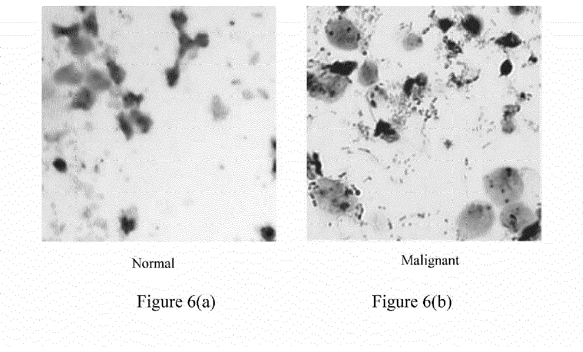

Figure 6(a) shows the expression of 9 imprinted genes in the exfoliated cells in stool from a healthy human; and Figure 6(b) shows the expression of 9 imprinted genes in the exfoliated cells in stool from a patient with colorectal cancer.

## DETAILED DESCRIPTION

The invention is defined by the appended claims.

In order to further explain the technical means adopted by the present invention and effects thereof, the technical solutions of the present invention will be further described below in connection with the accompanying drawings and by means of specific examples. However, the present invention is not limited to the scope of the Examples.

Genomic imprinting is a way of gene regulation in epigenetics, and is characterized by methylating an allele from a particular parent to express only one allele of a gene while leave the other silent. Such a gene is called an imprinted gene. Loss of imprinting is an epigenetic change in which the demethylation of an imprinted gene causes the silent allele to be activated and begin to express. Numerous studies have shown that this phenomenon (loss of imprinting) is common in various cancers and occurs earlier than the changes in cell and tissue morphology. In a sharp contrast, in healthy cells, the occurrence of loss of imprinting is less. Therefore, the methylation status of imprinted genes can be used as a pathological marker to analyze the abnormal state of cells using specific molecular detection techniques.

The model and device of the present disclosure present the expression of the imprinted gene with loss of imprinting in a sample from a patient with colorectal tumor in an intuitive manner. By *in situ* labeling of imprinted genes, objective, intuitive, early, and accurate detection of changes in imprinted genes is achieved, and a quantitative model can also be provided, making a significant contribution to the diagnosis of colorectal tumors.

The detection device of the present disclosure can be used to determine the benignity or malignancy of colorectal tumor from enteroscopy biopsy and exfoliated cells in stool before surgery for patients with colorectal tumor, so as to provide a basis for surgery and accurate treatment. This is a revolutionary breakthrough in the diagnosis of colorectal tumor in the field of cellular and molecular biology.

The present disclosure can be used to accurately distinguish colorectal adenomas from early adenocarcinomas, and greatly improve the early and accurate diagnosis of colorectal cancer by clearly grading the malignancy of colorectal tumor by combined detection of imprinted genes. In particular, exfoliated cells in stool are not invasive to patients, and are easily sampled, and can gain time and make a significant contribution to saving patients' lives when used for early mass screening and postoperative follow-up, especially for follow-up of patients suspected of recurrence.

The disclosure can be used to accurately determine the benignity or malignancy of tissues adjacent to colorectal cancer, guide the scope of resection in clinical surgery, and greatly reduce the postoperative recurrence of colorectal cancer.

The detection method of the present disclosure is different from the immunohistochemical method, reducing false positives and other negative effects. Moreover, by discovering the sites of colorectal tumor-associated imprinted genes with loss of imprinting, it is found that targeting drugs or methods leading to silencing, deletion, or rearrangement of the gene can be used to guide subsequent treatment and medication.

**Example 1 Imprinted Gene Analysis for Colorectal Cancer**

The method for detecting an imprinted gene includes the following steps:

(1) Tissue sections (10 $\mu$m) of colorectal cancer were obtained and fixed in 10% neutral buffered formalin solution to prevent RNA degradation for a fixation time of 24 hours. The sections were embedded in paraffin (FFPE method) and the slides used for this experiment were positive charged slides. The sections were heated in an oven at 40 °C for more than 3 hours.

(2) The sections were deparaffinized according to the sample processing method of RNAscope. The endogenous peroxidase activity in the sample was blocked, the sample was enhanced in permeability and RNA was exposed.

(3) Probe design: specific probes were designed according to the imprinted genes. The probes were designed according to imprinted genes Z1 (Gnas), Z4 (Igf2r), Z5 (Mest), Z6 (Plagl1), Z8 (Dcn), Z10 (Gatm), Z11 (Grb10), Z13 (Sgce), and Z16 (Snrpn/Snurf). Specifically, a sequence within an intron of each gene was selected for probe design. Specific probes were design by Advanced Cell Diagnostics.

(4) RNAscope *in situ* hybridization was performed on the samples using the probes of step (3) according to the protocol of the kit.

(5) Signal was amplified and the sections were stained with hematoxylin. The expressions of imprinted genes were analyzed through microscope images.

In model of the present disclosure, the formulas for calculating the expression of an imprinted gene, the expression of an imprinted gene with loss of imprinting and the expression of an imprinted gene with copy number variation are as follows:

$$\text{Total expression of an imprinted gene} = (b+c+d) \,/\, (a+b+c+d) \times 100\%;$$

$$\text{Expression of a normal imprinted gene} = b \,/\, (b+c+d) \times 100\%;$$

$$\text{Expression of an imprinted gene with loss of imprinting (LOI)} = c / (b+c+d) \times 100\%;$$

$$\text{Expression of an imprinted gene with copy number variation (CNV)} = d / (b+c+d) \times 100\%;$$

wherein a, b, c, and d are shown in Figure 1, and wherein "a" represents that after performing hematoxylin staining on a cell, there is no mark in the nucleus, the imprinted gene has no expression; "b" represents that after performing hematoxylin staining on a cell, there is one red/brown mark in the nucleus, the imprinted gene exists; "c" represents that after performing hematoxylin staining on a cell, there are two red/brown marks in the nucleus, the imprinted gene loses imprinting; and "d" represents that after performing hematoxylin staining on a cell, there are more than two red/brown marks in the nucleus, the imprinted gene has copy number variation.

As shown in figures 2(a)-2(e), from Grade 0 to Grade IV, the proportions of cells having an imprinted gene with loss of imprinting (there are two marks in one nucleus) and cells having an imprinted gene with copy number variation (there are three or more marks in one nucleus) increase with the malignancy of the tumor.

**Example 2 Imprinted Gene Analysis of Enteroscopy Biopsy Samples**

The enteroscopy biopsy sample was obtained by removing suspiciously diseased tissue under an endoscope, and fixing it in a 10% neutral buffered formalin solution for more than 24 hours. The other detection procedures were the same as those in Example 1.

As shown in figures 3 (a) to 3 (f), genes Z1, Z4, Z5, Z6, Z8, Z10, Z11, Z13 and Z16 each show different sensitivity in colorectal cancer, or different degree and state of loss of imprinting in colorectal cancer.

Specifically, the sensitivity of each imprinted gene to colorectal cancer is shown in figures 4(a)-4(i). As shown in figures 4(a)-4(d), the expression of the imprinted gene Z1 and imprinted gene Z1 with loss of imprinting begins to increase in the potential malignancy, and quickly rises to a very high level during the development of colorectal cancer, and the expression of the imprinted gene Z1 with copy number variation increases rapidly to a very high level during the potential malignancy, and further increases slightly from early to advanced colorectal cancer; the expression of the imprinted gene Z5 and imprinted gene Z5 with loss of imprinting begins to increase in the potential malignancy, quickly rises to a very high level in early colorectal cancer, and is maintained at the level in moderate and advanced colorectal cancer, and the expression of the imprinted gene Z5 with copy number variation occurs in the potential malignancy, and gradually rise to a very high level during the development from early to advanced colorectal cancer; the expression of the imprinted gene Z10 with loss of imprinting occurs in the potential malignancy, and does not increase significantly in early colorectal cancer, and however gradually rises to a very high level in moderate and advanced colorectal cancer, the expression of imprinted gene Z10 and imprinted gene Z10 with copy number variation increases rapidly in the potential malignancy and early colorectal cancer, increase slowly in the moderate colorectal cancer, and again increases rapidly to a very high level in the advanced colorectal cancer; the expression of the imprinted gene Z11 with loss of imprinting and imprinted gene Z11 with copy number variation increases rapidly in the potential malignancy, and further increases to a very high level during the development from early to advanced colorectal cancer, and the expression of imprinted gene Z11 begins to increase in the potential malignancy, and gradually rises to a very high level during the development of colorectal cancer.

As shown in figures 4 (e)-4 (i), the expression of the imprinted gene Z4 with loss of imprinting does not increase significantly from potential malignancy to moderate colorectal cancer, and however rapidly rises to a higher level in advanced colorectal cancer, and the expression of the imprinted gene Z4 with copy number variation occurs in potential malignancy, slowly increases in from early to moderate colorectal cancer, and quickly rises to a higher level in advanced colorectal cancer, and the expression of the imprinted gene Z4 begins to increase in potential malignancy, rapid rises in early colorectal cancer, then slowly rises in moderate colorectal cancer, and again quickly rises to a higher level in advanced colorectal cancer; the expression of the imprinted gene Z6 with loss of imprinting, of the imprinted gene Z6 with copy number variation and of the imprinted gene Z6 begins to increase in potential malignancy, and gradually rises to a higher level with the development of colorectal cancer; the expression of the imprinted gene Z8 with loss of imprinting, of the imprinted gene Z8 with copy number variation and of the imprinted gene Z8 increases rapidly in early colorectal cancer, and however slowly increase in moderate and advanced colorectal cancer; the expression of the imprinted gene Z13 with loss of imprinting, of the imprinted gene Z13 with copy number variation and of the imprinted gene Z13 increase rapidly during the potential malignancy, and however stops increasing from early to advanced colorectal cancer; the expression of the imprinted gene Z16 with loss of imprinting, of the imprinted gene Z16 with copy number variation and of the imprinted gene Z16 rapidly increases in potential malignancy, remains stable in early colorectal cancer, rise rapidly in moderate colorectal cancer, and then slowly rises in advanced colorectal cancer.

**Example 3 Imprinted Gene Analysis of 107 Colorectal Tumor Samples**

Tissues including enteroscopy biopsy samples were obtained from 107 patients with colorectal tumor. The detection procedure was the same as that in Example 1.

As shown in figures 5(a)-5(i), the ratio of imprinted gene with loss of imprinting and imprinted gene with copy number variation of the nine probes among tissue samples from 107 colorectal tumors showed a distribution trend from low to high. According to the distribution trend of different probes, the grading standards shown by the dotted line in the figures were calculated. The imprinted gene with loss of imprinting and the imprinted gene with copy number variation of each probe are divided into five grades from low to high, respectively, and the specific grades are classified as follows:

As shown in Figure 5(a), for the imprinted gene Z1, Grade 0 is defined as any one or more of: the expression of the imprinted gene with loss of imprinting of less than 15%, the expression of the imprinted gene with copy number variation of less than 0.8%, or the total expression of the imprinted gene of less than 20%; Grade I is defined as any one or more of: the expression of the imprinted gene with loss of imprinting of 15-20%, the expression of the imprinted gene with copy number variation of 0.8-2%, or the total expression of the imprinted gene of 20-30%; Grade II is defined as any one or more of: the expression of the imprinted gene with loss of imprinting of 20-25%, the expression of the imprinted gene with copy number variation of 2-3.5%, or the total expression of the imprinted gene of 30-40%; Grade III is defined as any one or more of: the expression of the imprinted gene with loss of imprinting of 25-30%, the expression of the imprinted gene with copy number variation of 3.5-5%, or the total expression of the imprinted gene of 40-50%; and Grade IV is defined as any one or more of: the expression of the imprinted gene with loss of imprinting of more than 30%, the expression of the imprinted gene with copy number variation of more than 5%, or the total expression of the imprinted gene of more than 50%.

As shown in Figure 5(b), for the imprinted gene Z5, Grade 0 is defined as any one or more of: the expression of the imprinted gene with loss of imprinting of less than 10%, the expression of the imprinted gene with copy number variation of less than 0.8%, or the total expression of the imprinted gene of less than 15%; Grade I is defined as any one or more of: the expression of the imprinted gene with loss of imprinting of 10-20%, the expression of the imprinted gene with copy number variation of 0.8-1.5%, or the total expression of the imprinted gene of 15-20%; Grade II is defined as any one or more of: the expression of the imprinted gene with loss of imprinting of 20-25%, the expression of the imprinted gene with copy number variation of 1.5-3%, or the total expression of the imprinted gene of 20-30%; Grade III is defined as any one or more of: the expression of the imprinted gene with loss of imprinting of 25-30%, the expression of the imprinted gene with copy number variation of 3-5%, or the total expression of the imprinted gene of 30-40%; and Grade IV is defined as any one or more of: the expression of the imprinted gene with loss of imprinting of more than 30%, the expression of the imprinted gene with copy number variation of more than 5%, or the total expression of the imprinted gene of more than 40%.

As shown in Figure 5(c), for the imprinted gene Z10, Grade 0 is defined as any one or more of: the expression of the imprinted gene with loss of imprinting of less than 10%, the expression of the imprinted gene with copy number variation of less than 0.8%, or the total expression of the imprinted gene of less than 15%; Grade I is defined as any one or more of: the expression of the imprinted gene with loss of imprinting of 10-20%, the expression of the imprinted gene with copy number variation of 0.8-1.5%, or the total expression of the imprinted gene of 15-20%; Grade II is defined as any one or more of: the expression of the imprinted gene with loss of imprinting of 20-25%, the expression of the imprinted gene with copy number variation of 1.5-3%, or the total expression of the imprinted gene of 20-30%; Grade III is defined as any one or more of: the expression of the imprinted gene with loss of imprinting of 25-30%, the expression of the imprinted gene with copy number variation of 3-5%, or the total expression of the imprinted gene of 30-40%; and Grade IV is defined as any one or more of: the expression of the imprinted gene with loss of imprinting of more than 30%, the expression of the imprinted gene with copy number variation of more than 5%, or the total expression of the imprinted gene of more than 40%.

As shown in Figure 5(d), for the imprinted gene Z11, Grade 0 is defined as any one or more of: the expression of the imprinted gene with loss of imprinting of less than 15%, the expression of the imprinted gene with copy number variation of less than 0.8%, or the total expression of the imprinted gene of less than 20%; Grade I is defined as any one or more of: the expression of the imprinted gene with loss of imprinting of 15-20%, the expression of the imprinted gene with copy number variation of 0.8-2%, or the total expression of the imprinted gene of 20-30%; Grade II is defined as any one or more of: the expression of the imprinted gene with loss of imprinting of 20-25%, the expression of the imprinted gene with copy number variation of 2-3.5%, or the total expression of the imprinted gene of 30-40%; Grade III is defined as any one or more of: the expression of the imprinted gene with loss of imprinting of 25-30%, the expression of the imprinted gene with copy number variation of 3.5-5%, or the total expression of the imprinted gene of 40-50%;

and Grade IV is defined as any one or more of: the expression of the imprinted gene with loss of imprinting of more than 30%, the expression of the imprinted gene with copy number variation of more than 5%, or the total expression of the imprinted gene of more than 50%.

As shown in Figure 5(e), for the imprinted gene Z4, Grade 0 is defined as any one or more of: the expression of the imprinted gene with loss of imprinting of less than 10%, the expression of the imprinted gene with copy number variation of less than 0.8%, or the total expression of the imprinted gene of less than 15%; Grade I is defined as any one or more of: the expression of the imprinted gene with loss of imprinting of 10-20%, the expression of the imprinted gene with copy number variation of 0.8-1.5%, or the total expression of the imprinted gene of 15-20%; Grade II is defined as any one or more of: the expression of the imprinted gene with loss of imprinting of 20-25%, the expression of the imprinted gene with copy number variation of 1.5-3%, or the total expression of the imprinted gene of 20-30%; Grade III is defined as any one or more of: the expression of the imprinted gene with loss of imprinting of 25-30%, the expression of the imprinted gene with copy number variation of 3-5%, or the total expression of the imprinted gene of 30-40%; and Grade IV is defined as any one or more of: the expression of the imprinted gene with loss of imprinting of more than 30%, the expression of the imprinted gene with copy number variation of more than 5%, or the total expression of the imprinted gene of more than 40%.

As shown in Figure 5(f), for the imprinted gene Z6, Grade 0 is defined as any one or more of: the expression of the imprinted gene with loss of imprinting of less than 10%, the expression of the imprinted gene with copy number variation of less than 0.8%, or the total expression of the imprinted gene of less than 15%; Grade I is defined as any one or more of: the expression of the imprinted gene with loss of imprinting of 10-20%, the expression of the imprinted gene with copy number variation of 0.8-1.5%, or the total expression of the imprinted gene of 15-20%; Grade II is defined as any one or more of: the expression of the imprinted gene with loss of imprinting of 20-25%, the expression of the imprinted gene with copy number variation of 1.5-3%, or the total expression of the imprinted gene of 20-30%; Grade III is defined as any one or more of: the expression of the imprinted gene with loss of imprinting of 25-30%, the expression of the imprinted gene with copy number variation of 3-5%, or the total expression of the imprinted gene of 30-40%; and Grade IV is defined as any one or more of: the expression of the imprinted gene with loss of imprinting of more than 30%, the expression of the imprinted gene with copy number variation of more than 5%, or the total expression of the imprinted gene of more than 40%.

As shown in Figure 5(g), for the imprinted gene Z8, Grade 0 is defined as any one or more of: the expression of the imprinted gene with loss of imprinting of less than 10%, the expression of the imprinted gene with copy number variation of less than 0.8%, or the total expression of the imprinted gene of less than 15%; Grade I is defined as any one or more of: the expression of the imprinted gene with loss of imprinting of 10-20%, the expression of the imprinted gene with copy number variation of 0.8-1.5%, or the total expression of the imprinted gene of 15-20%; Grade II is defined as any one or more of: the expression of the imprinted gene with loss of imprinting of 20-25%, the expression of the imprinted gene with copy number variation of 1.5-3%, or the total expression of the imprinted gene of 20-30%; Grade III is defined as any one or more of: the expression of the imprinted gene with loss of imprinting of 25-30%, the expression of the imprinted gene with copy number variation of 3-5%, or the total expression of the imprinted gene of 30-40%; and Grade IV is defined as any one or more of: the expression of the imprinted gene with loss of imprinting of more than 30%, the expression of the imprinted gene with copy number variation of more than 5%, or the total expression of the imprinted gene of more than 40%.

As shown in Figure 5(h), for the imprinted gene Z13, Grade 0 is defined as any one or more of: the expression of the imprinted gene with loss of imprinting of less than 10%, the expression of the imprinted gene with copy number variation of less than 0.8%, or the total expression of the imprinted gene of less than 15%; Grade I is defined as any one or more of: the expression of the imprinted gene with loss of imprinting of 10-20%, the expression of the imprinted gene with copy number variation of 0.8-1.5%, or the total expression of the imprinted gene of 15-20%; Grade II is defined as any one or more of: the expression of the imprinted gene with loss of imprinting of 20-25%, the expression of the imprinted gene with copy number variation of 1.5-3%, or the total expression of the imprinted gene of 20-30%; Grade III is defined as any one or more of: the expression of the imprinted gene with loss of imprinting of 25-30%, the expression of the imprinted gene with copy number variation of 3-5%, or the total expression of the imprinted gene of 30-40%; and Grade IV is defined as any one or more of: the expression of the imprinted gene with loss of imprinting of more than 30%, the expression of the imprinted gene with copy number variation of more than 5%, or the total expression of the imprinted gene of more than 40%.

As shown in Figure 5(i), for the imprinted gene Z16, Grade 0 is defined as any one or more of: the expression of the imprinted gene with loss of imprinting of less than 15%, the expression of the imprinted gene with copy number variation of less than 0.8%, or the total expression of the imprinted gene of less than 20%; Grade I is defined as any one or more of: the expression of the imprinted gene with loss of imprinting of 15-20%, the expression of the imprinted gene with copy number variation of 0.8-2%, or the total expression of the imprinted gene of 20-30%; Grade II is defined as any one or more of: the expression of the imprinted gene with loss of imprinting of 20-25%, the expression of the imprinted gene with copy number variation of 2-3.5%, or the total expression of the imprinted gene of 30-40%; Grade III is defined as any one or more of: the expression of the imprinted gene with loss of imprinting of 25-30%, the expression of the imprinted gene with copy number variation of 3.5-5%, or the total expression of the imprinted gene of 40-50%; and Grade IV is defined as any one or more of: the expression of the imprinted gene with loss of imprinting of more than 30%, the expression of the imprinted gene with copy number variation of more than 5%, or the total expression of the imprinted gene of more than 50%.

From the comprehensive analysis of these 107 colorectal tumor samples, it can be concluded that:

The benignity or malignancy of the colorectal tumor is classified as benign tumor, potential colorectal cancer, early colorectal cancer, moderate colorectal cancer, or advanced colorectal cancer;

The colorectal tumor is determined as a benign tumor, if the expression of the imprinted genes Z1, Z4, Z5, Z6, Z8, Z10, Z11, Z13 and Z16 with loss of imprinting and the expression of the imprinted genes Z1, Z4, Z5, Z6, Z8, Z10, Z11, Z13 and Z16 with copy number variation both are less than Grade I, or if the expression of no more than one of the imprinted genes Z1, Z4, Z5, Z6, Z8, Z10, Z11, Z13 and Z16 with loss of imprinting is Grade I and the expression of no more than one of the imprinted genes Z1, Z4, Z5, Z6, Z8, Z10, Z11, Z13 and Z16 with copy number variation is Grade I;

The colorectal tumor is determined as a potential colorectal cancer, if the expression of at least two of the imprinted genes Z1, Z4, Z5, Z6, Z8, Z10, Z11, Z13 and Z16 with loss of imprinting is Grade I, if the expression of at least two of the imprinted genes Z1, Z4, Z5, Z6, Z8, Z10, Z11, Z13 and Z16 with copy number variation is Grade I, or if the expression of no more than one of the imprinted genes Z1, Z4, Z5, Z6, Z8, Z10, Z11, Z13 and Z16 with loss of imprinting is Grade II and the expression of no more than one of the imprinted genes Z1, Z4, Z5, Z6, Z8, Z10, Z11, Z13 and Z16 with copy number variation is Grade II;

The colorectal tumor is determined as an early colorectal cancer, if the expression of at least two of the imprinted genes Z1, Z4, Z5, Z6, Z8, Z10, Z11, Z13 and Z16 with loss of imprinting is Grade II, if the expression of at least two of the imprinted genes Z1, Z4, Z5, Z6, Z8, Z10, Z11, Z13 and Z16 with copy number variation is Grade II, or if the expression of no more than one of the imprinted genes Z1, Z4, Z5, Z6, Z8, Z10, Z11, Z13 and Z16 with loss of imprinting is Grade III and the expression of no more than one of the imprinted genes Z1, Z4, Z5, Z6, Z8, Z10, Z11, Z13 and Z16 with copy number variation is Grade III;

The colorectal tumor is determined as a moderate colorectal cancer, if the expression of at least two of the imprinted genes Z1, Z4, Z5, Z6, Z8, Z10, Z11, Z13 and Z16 with loss of imprinting is Grade III, if the expression of at least two of the imprinted genes Z1, Z4, Z5, Z6, Z8, Z10, Z11, Z13 and Z16 with copy number variation is Grade III, or if the expression of no more than one of the imprinted genes Z1, Z4, Z5, Z6, Z8, Z10, Z11, Z13 and Z16 with loss of imprinting is Grade IV and the expression of no more than one of the imprinted genes Z1, Z4, Z5, Z6, Z8, Z10, Z11, Z13 and Z16 with copy number variation is Grade IV; or

The colorectal tumor is determined as an advanced colorectal cancer, if the expression of at least two of the imprinted genes Z1, Z4, Z5, Z6, Z8, Z10, Z11, Z13 and Z16 with loss of imprinting is Grade IV, or if the expression of at least two of the imprinted genes Z1, Z4, Z5, Z6, Z8, Z10, Z11, Z13 and Z16 with copy number variation is Grade IV

## Example 4 Imprinted Gene Analysis of Exfoliated Cells in Stool

Intestinally exfoliated cells in fresh stool were obtained and fixed in 10% neutral buffered formalin for more than 24 hours. Other detection procedures were the same as in Example 1. The results are shown in figures 6(a)-6(b).

As shown in figures 6 (a) to 6 (b), figure 6 (a) shows the exfoliated cells in stool from a healthy human, and figure 6 (b) shows the exfoliated cells in stool from a patient with colorectal cancer. Most of the imprinted genes in the exfoliated cells in stool from a healthy human are not expressed or are normally expressed, and the expression of an imprinted gene with loss of imprinting or with copy number variation is present in a large proportion of the exfoliated cells in stool from a patient with colorectal cancer.

In summary, the model and device of the present disclosure present the characteristic of the imprinted gene with loss of imprinting on the colorectal tumor samples in an intuitive manner. By *in situ* labeling of imprinted genes, objective, intuitive, early, and accurate detection of changes in imprinted genes is achieved, and a quantitative model is also provided, making a significant contribution to the diagnosis of colorectal tumors.

The Applicant declares that the present application illustrates the detailed device of the present invention by the above examples, but not limited to the above detailed examples.

## Claims

1. A device for determining the benignity or malignancy of a colorectal tumor, comprising the following units:

    (1) sample unit for obtaining a test sample;
    (2) probe design unit for designing a probe specific for the sequence of an imprinted gene, wherein the probe is a sequence within an intron of the imprinted gene;

(3) detection unit for performing *in situ* hybridization of the probe of step (2) to the test sample;

(4) analysis unit for analyzing microscopic images and determining the expression level of the imprinted gene; wherein, the analysis unit is operated by calculating the expression of the imprinted gene with loss of imprinting, the expression of the imprinted gene with copy number variation and the total expression of the imprinted gene, and grading the expression of the imprinted gene with loss of imprinting and the expression of the imprinted gene with copy number variation, to grade the expression of the imprinted gene;

wherein the imprinted gene is any one or more of Z1, Z4, Z5, Z6, Z8, Z10, Z11, Z13 or Z16, and wherein the imprinted gene Z1 is Gnas, the imprinted gene Z5 is Mest, the imprinted gene Z10 is Gatm, the imprinted gene Z11 is Grb10, the imprinted gene Z4 is Igf2r, the imprinted gene Z6 is Plagl1, the imprinted gene Z8 is Dcn, the imprinted gene Z13 is Sgce, and the imprinted gene Z16 is Snrpn/Snurf;

wherein the total expression of an imprinted gene, the expression of an imprinted gene with loss of imprinting and the expression of an imprinted gene with copy number variation are calculated by the following formulas:

$$\text{Total expression of an imprinted gene} = (b+c+d) / (a+b+c+d) \times 100\%;$$

$$\text{Expression of a normal imprinted gene} = b / (b+c+d) \times 100\%;$$

$$\text{Expression of an imprinted gene with loss of imprinting} = c / (b+c+d) \times 100\%;$$

$$\text{Expression of an imprinted gene with copy number variation} = d / (b+c+d) \times 100\%;$$

wherein,

a represents that after performing hematoxylin staining on a cell, there is no mark in the nucleus of the cell, the imprinted gene has no expression;

b represents that after performing hematoxylin staining on a cell, there is one red/brown mark in the nucleus of the cell, the imprinted gene exists;

c represents that after performing hematoxylin staining on a cell, there are two red/brown marks in the nucleus of the cell, the imprinted gene loses imprinting; and

d represents that after performing hematoxylin staining on a cell, there are more than two red/brown markers in the nucleus of the cell, the imprinted gene has an copy number variation;

wherein the expression of Z 1, Z11 or Z16 with loss of imprinting, the expression of Z1, Z11 or Z16 with copy number variation, and the total expression of Z1, Z11 or Z16 are classified into 5 grades:

Grade 0: any one or more of: the expression of Z1, Z11 or Z16 with loss of imprinting of less than 15%, the expression of Z1, Z11 or Z16 with copy number variation of less than 0.8%, or the total expression of Z1, Z11 or Z16 of less than 20%;

Grade I: any one or more of: the expression of Z1, Z11 or Z16 with loss of imprinting of 15-20%, the expression of Z1, Z11 or Z16 with copy number variation of 0.8-2%, or the total expression of Z1, Z11 or Z16 of 20-30%;

Grade II: any one or more of: the expression of Z1, Z11 or Z16 with loss of imprinting of 20-25%, the expression of Z1, Z11 or Z16 with copy number variation of 2-3.5%, or the total expression of Z 1, Z11 or Z16 of 30-40%;

Grade III: any one or more of: the expression of Z1, Z11 or Z16 with loss of imprinting of 25-30%, the expression of Z1, Z11 or Z16 with copy number variation of 3.5-5%, or the total expression of Z1, Z11 or Z16 of 40-50%; and

Grade IV: any one or more of: the expression of Z1, Z11 or Z16 with loss of imprinting of more than 30%, the expression of Z1, Z11 or Z16 with copy number variation of more than 5%, or the total expression of Z1, Z11 or Z16 of more than 50%; and

wherein the expression of Z4, Z5, Z6, Z8, Z10 or Z13 with loss of imprinting, the expression of Z4, Z5, Z6, Z8, Z10 or Z13 with copy number variation, and the total expression of Z4, Z5, Z6, Z8, Z10 or Z13 are classified into 5 grades:

Grade 0: any one or more of: the expression of Z4, Z5, Z6, Z8, Z10 or Z13 with loss of imprinting of less than 10%, the expression of Z4, Z5, Z6, Z8, Z10 or Z13 with copy number variation of less than 0.8%, or the total expression of Z4, Z5, Z6, Z8, Z10 or Z13 of less than 15%;

Grade I: any one or more of: the expression of Z4, Z5, Z6, Z8, Z10 or Z13 with loss of imprinting of 10-20%, the expression of Z4, Z5, Z6, Z8, Z10 or Z13 with copy number variation of 0.8-1.5%, or the total expression of Z4, Z5, Z6, Z8, Z10 or Z13 of 15-20%;

Grade II: any one or more of: the expression of Z4, Z5, Z6, Z8, Z10 or Z13 with loss of imprinting of 20-25%, the expression of Z4, Z5, Z6, Z8, Z10 or Z13 with copy number variation of 1.5-3%, or the total expression of Z4, Z5, Z6, Z8, Z10 or Z13 of 20-30%;

Grade III: any one or more of: the expression of Z4, Z5, Z6, Z8, Z10 or Z13 with loss of imprinting of 25-30%, the expression of Z4, Z5, Z6, Z8, Z10 or Z13 with copy number variation of 3-5%, or the total expression of Z4, Z5, Z6, Z8, Z10 or Z13 of 30-40%; and

Grade IV: any one or more of: the expression of Z4, Z5, Z6, Z8, Z10 or Z13 with loss of imprinting of more than 30%, the expression of Z4, Z5, Z6, Z8, Z10 or Z13 with copy number variation of more than 5%, or the total expression of Z4, Z5, Z6, Z8, Z10 or Z13 of more than 40%; and wherein:

the colorectal tumor is determined as a benign tumor, if the expression of the imprinted genes Z1, Z4, Z5, Z6, Z8, Z10, Z11, Z13 and Z16 with loss of imprinting and the expression of the imprinted genes Z1, Z4, Z5, Z6, Z8, Z10, Z11, Z13 and Z16 with copy number variation both are less than Grade I, or if the expression of no more than one of the imprinted genes Z1, Z4, Z5, Z6, Z8, Z10, Z11, Z13 and Z16 with loss of imprinting is Grade I and the expression of no more than one of the imprinted genes Z1, Z4, Z5, Z6, Z8, Z10, Z11, Z13 and Z16 with copy number variation is Grade I;

the colorectal tumor is determined as a potential colorectal cancer, if the expression of at least two of the imprinted genes Z1, Z4, Z5, Z6, Z8, Z10, Z11, Z13 and Z16 with loss of imprinting is Grade I, if the expression of at least two of the imprinted genes Z1, Z4, Z5, Z6, Z8, Z10, Z11, Z13 and Z16 with copy number variation is Grade I, or if the expression of no more than one of the imprinted genes Z1, Z4, Z5, Z6, Z8, Z10, Z11, Z13 and Z16 with loss of imprinting is Grade II and the expression of no more than one of the imprinted genes Z1, Z4, Z5, Z6, Z8, Z10, Z11, Z13 and Z16 with copy number variation is Grade II;

the colorectal tumor is determined as an early colorectal cancer, if the expression of at least two of the imprinted genes Z1, Z4, Z5, Z6, Z8, Z10, Z11, Z13 and Z16 with loss of imprinting is Grade II, if the expression of at least two of the imprinted genes Z1, Z4, Z5, Z6, Z8, Z10, Z11, Z13 and Z16 with copy number variation is Grade II, or if the expression of no more than one of the imprinted genes Z1, Z4, Z5, Z6, Z8, Z10, Z11, Z13 and Z16 with loss of imprinting is Grade III and the expression of no more than one of the imprinted genes Z1, Z4, Z5, Z6, Z8, Z10, Z11, Z13 and Z16 with copy number variation is Grade III;

the colorectal tumor is determined as a moderate colorectal cancer, if the expression of at least two of the imprinted genes Z1, Z4, Z5, Z6, Z8, Z10, Z11, Z13 and Z16 with loss of imprinting is Grade III, if the expression of at least two of the imprinted genes Z1, Z4, Z5, Z6, Z8, Z10, Z11, Z13 and Z16 with copy number variation is Grade III, or if the expression of no more than one of the imprinted genes Z1, Z4, Z5, Z6, Z8, Z10, Z11, Z13 and Z16 with loss of imprinting is Grade IV and the expression of no more than one of the imprinted genes Z1, Z4, Z5, Z6, Z8, Z10, Z11, Z13 and Z16 with copy number variation is Grade IV; or

the colorectal tumor is determined as an advanced colorectal cancer, if the expression of at least two of the imprinted genes Z1, Z4, Z5, Z6, Z8, Z10, Z11, Z13 and Z16 with loss of imprinting is Grade IV, or if the expression of at least two of the imprinted genes Z1, Z4, Z5, Z6, Z8, Z10, Z11, Z13 and Z16 with copy number variation is Grade IV

2. The device according to claim 1, wherein the calculation of the imprinted gene expression comprises calculating the expression of any one of Z1, Z5, Z10 or Z11, preferably Z1 or Z11, and more preferably Z11.

3. The device according to claim 1, wherein the calculation of the imprinted gene expression comprises calculating the expression of a combination of any two imprinted genes of Z1, Z5, Z10 or Z11, preferably a combination of Z1 and Z11 or a combination of Z1 and Z5.

**4.** The device according to claim 1, wherein the calculation of the imprinted gene expression comprises calculating the expression of a combination of imprinted genes, wherein the combination is a combination of nine imprinted genes Z1, Z4, Z5, Z6, Z8, Z10, Z11, Z13 and Z16.

**5.** The device according to claim 1, wherein the test sample of step (1) is derived from human tissue and/or cells.

**6.** The device according to claim 5, wherein the test sample is a tissue paraffin section, an enteroscopy biopsy sample and/or exfoliated cells in stool.

**7.** The device according to claim 1, wherein the *in situ* hybridization is RNAscope *in situ* hybridization.

**8.** The device according to claim 7, wherein the RNAscope *in situ* hybridization is performed by using singleplex or multiplex color assay kit or singleplex or multiplex fluorescence assay kit, preferably singleplex red/brown color assay kit or multiplex fluorescence assay kit.

**9.** Use of the device according to claim 1 for detection of a colorectal tumor.


**Patentansprüche**

**1.** Eine Vorrichtung zur Bestimmung der Gutartigkeit oder Bösartigkeit eines kolorektalen Tumors umfassend die folgenden Einheiten:

> (1) Sammeleinheit zum Erhalt einer Testprobe;
> (2) Sonden-Design-Einheit zum Designen einer Sonde, die für die Sequenz eines geprägten (imprinted) Gens spezifisch ist, wobei die Sonde eine Sequenz innerhalb eines Introns des geprägten Gens ist;
> (3) Detektionseinheit zum Durchführen von *in situ* Hybridisierung der Sonde aus Schritt (2) an die Testprobe;
> (4) Analyseeinheit zum Analysieren von mikroskopischen Bildern und Bestimmung des Expressionslevels des geprägten Gens;
> wobei die Analyseeinheit durch die Kalkulation der Expression des geprägten Gens mit Verlust der Prägung (des Imprintings), der Expression des geprägten Gens mit Abweichung der Kopienanzahl und der Gesamtexpression des geprägten Gens und der Bewertung der Expression des geprägten Gens mit Verlust der Prägung und der Expression des geprägten Gens mit Abweichung der Kopienzahl betrieben wird, um die Expression des geprägten Gens zu bewerten;
> wobei das geprägte Gen eines oder mehrere von Z1, Z4, Z5, Z6, Z8, Z10, Z11, Z13 oder Z16 ist und wobei das geprägte Gen Z1 Gnas ist, das geprägte Gen Z5 Mest ist, das geprägte Gen Z10 Gatm ist, das geprägte Gen Z11 Grb10 ist, das geprägte Gen Z4 Igf2r ist, das geprägte Gen Z6 Plagl1 ist, das geprägte Gen Z8 Den ist, das geprägte GenZ13 Sgce ist und das geprägte Gen Z16 Snrpn/Snurf ist;
> wobei die Gesamtexpression eines geprägten Gens, die Expression eines geprägten Gens mit Verlust der Prägung und die Expression eines geprägten Gens mit Abweichung der Kopienzahl anhand der folgenden Formeln kalkuliert werden:

$$\text{Gesamtexpression eines geprägten Gens} = (b+c+d)/(a+b+c+d) \times 100\%;$$

$$\text{Expression eines normalen geprägten Gens} = b/(b+c+d) \times 100\%;$$

$$\text{Expression eines geprägten Gens mit Verlust der Prägung} = c/(b+c+d) \times 100\%;$$

$$\text{Expression eines geprägten Gens mit Abweichung der Kopienzahl} = d/(b+c+d) \times 100\%;$$

> wobei,

a repräsentiert, dass nach Durchführung der Hämatoxylin-Färbung auf einer Zelle keine Markierung im Nukleus der Zelle ist, dass das geprägte Gen keine Expression hat;

b repräsentiert, dass nach Durchführung der Hämatoxylin-Färbung auf einer Zelle eine rote/braune Markierung im Nukleus der Zelle ist, dass das geprägte Gen existiert;

c repräsentiert, dass nach Durchführung der Hämatoxylin-Färbung auf einer Zelle zwei rote/braune Markierungen im Nukleus der Zelle sind, dass das geprägte Gen die Prägung verliert; und

d repräsentiert, dass nach Durchführung der Hämatoxylin-Färbung auf einer Zelle mehr als zwei rote/braune Markierungen im Nukleus der Zelle sind, dass das geprägte Gen eine Abweichung der Kopienzahl hat;

wobei die Expression von Z1, Z11 oder Z16 mit Verlust der Prägung, die Expression von Z1, Z11 oder Z16 mit Abweichung der Kopienzahl und die Gesamtexpression von Z1, Z11 oder Z16 in 5 Stufen klassifiziert werden:

Stufe 0: eines oder mehrere von: die Expression von Z1, Z11 oder Z16 mit Verlust der Prägung von weniger als 15%, die Expression von Z1, Z11 oder Z16 mit Abweichung der Kopienzahl von weniger als 0,8% oder die Gesamtexpression von Z1, Z11 oder Z16 von weniger als 20%;

Stufe I: eines oder mehrere von: die Expression von Z1, Z11 oder Z16 mit Verlust der Prägung von 15-20%, die Expression von Z1, Z11 oder Z16 mit Abweichung der Kopienzahl von 0,8-2% oder die Gesamtexpression von Z1, Z11 oder Z16 von 20-30%;

Stufe II: eines oder mehrere von: die Expression von Z1, Z11 oder Z16 mit Verlust der Prägung von 20-25%, die Expression von Z1, Z11 oder Z16 mit Abweichung der Kopienzahl von 2-3,5% oder die Gesamtexpression von Z1, Z11 oder Z16 von 30-40%;

Stufe III: eines oder mehrere von: die Expression von Z1, Z11 oder Z16 mit Verlust der Prägung von 25-30%, die Expression von Z1, Z11 oder Z16 mit Abweichung der Kopienzahl von 3,5-5% oder die Gesamtexpression von Z1, Z11 oder Z16 von 40-50%; und

Stufe IV: eines oder mehrere von: die Expression von Z1, Z11 oder Z16 mit Verlust der Prägung von mehr als 30%, die Expression von Z1, Z11 oder Z16 mit Abweichung der Kopienzahl von mehr als 5% oder die Gesamtexpression von Z1, Z11 oder Z16 von mehr als 50%; und

wobei die Expression von Z4, Z5, Z6, Z8, Z10 oder Z13 mit Verlust der Prägung, die Expression von Z4, Z5, Z6, Z8, Z10 oder Z13 mit Abweichung der Kopienzahl und die Gesamtexpression von Z4, Z5, Z6, Z8, Z10 oder Z13 in 5 Stufen klassifiziert werden:

Stufe 0: eines oder mehrere von: die Expression von Z4, Z5, Z6, Z8, Z10 oder Z13 mit Verlust der Prägung von weniger als 10%, die Expression von Z4, Z5, Z6, Z8, Z10 oder Z13 mit Abweichung der Kopienzahl von weniger als 0,8% oder die Gesamtexpression von Z4, Z5, Z6, Z8, Z10 oder Z13 von weniger als 15%;

Stufe I: eines oder mehrere von: die Expression von Z4, Z5, Z6, Z8, Z10 oder Z13 mit Verlust der Prägung von 10-20%, die Expression von Z4, Z5, Z6, Z8, Z10 oder Z13 mit Abweichung der Kopienzahl von 0,8-1,5% oder die Gesamtexpression von Z4, Z5, Z6, Z8, Z10 oder Z13 von 15-20%;

Stufe II: eines oder mehrere von: die Expression von Z4, Z5, Z6, Z8, Z10 oder Z13 mit Verlust der Prägung von 20-25%, die Expression von Z4, Z5, Z6, Z8, Z10 oder Z13 mit Abweichung der Kopienzahl von 1,5-3% oder die Gesamtexpression von Z4, Z5, Z6, Z8, Z10 oder Z13 von 20-30%;

Stufe III: eines oder mehrere von: die Expression von Z4, Z5, Z6, Z8, Z10 oder Z13 mit Verlust der Prägung von 25-30%, die Expression von Z4, Z5, Z6, Z8, Z10 oder Z13 mit Abweichung der Kopienzahl von 3-5% oder die Gesamtexpression von Z4, Z5, Z6, Z8, Z10 oder Z13 von 30-40%; und

Stufe IV: eines oder mehrere von: die Expression von Z4, Z5, Z6, Z8, Z10 oder Z13 mit Verlust der Prägung von mehr als 30%, die Expression von Z4, Z5, Z6, Z8, Z10 oder Z13 mit Abweichung der Kopienzahl von mehr als 5% oder die Gesamtexpression von Z4, Z5, Z6, Z8, Z10 oder Z13 von mehr als 40%; und

wobei:

der kolorektale Tumor als ein gutartiger Tumor bewertet wird, wenn die Expression der geprägten Gene Z1, Z4, Z5, Z6, Z8, Z10, Z11, Z13 und Z16 mit Verlust der Prägung und die Expression der geprägten Gene Z1, Z4, Z5, Z6, Z8, Z10, Z11, Z13 und Z16 mit Abweichung der Kopienzahl beide weniger als Stufe 1 sind, oder wenn die Expression von nicht mehr als einem der geprägten Gene Z1, Z4, Z5, Z6, Z8, Z10, Z11, Z13 und Z16 mit Verlust der Prägung Stufe I ist und die Expression von nicht mehr als einem der geprägten Gene Z1, Z4, Z5, Z6, Z8, Z10, Z11, Z13 und Z16 mit Abweichung der Kopienzahl Stufe I ist;

der kolorektale Tumor als ein potenzieller kolorektaler Krebs bewertet wird, wenn die Expression von mindestens zwei der geprägten Gene Z1, Z4, Z5, Z6, Z8, Z10, Z11, Z13 und Z16 mit Verlust der Prägung Stufe I ist, wenn die Expression von mindestens zwei der geprägten Gene Z1, Z4, Z5, Z6, Z8, Z10, Z11, Z13 und Z16 mit Abweichung der Kopienzahl Stufe I ist, oder wenn die Expression von nicht mehr als einem der geprägten Gene Z1, Z4, Z5, Z6, Z8, Z10, Z11, Z13 und Z16 mit Verlust der Prägung Stufe II ist und die Expression von nicht mehr als einem der geprägten Gene Z1, Z4, Z5, Z6, Z8, Z10, Z11, Z13 und Z16 mit Abweichung der Kopienzahl Stufe II ist; der kolorektale Tumor als ein früher kolorektaler Krebs bewertet wird, wenn die Expression von mindestens zwei der geprägten Gene Z1, Z4, Z5, Z6, Z8, Z10, Z11, Z13 und Z16 mit Verlust der Prägung Stufe II ist, wenn die Expression von mindestens zwei der geprägten Gene Z1, Z4, Z5, Z6, Z8, Z10, Z11, Z13 und Z16 mit Abweichung der Kopienzahl Stufe II ist, oder wenn die Expression von nicht mehr als einem der geprägten Gene Z1, Z4, Z5, Z6, Z8, Z10, Z11, Z13 und Z16 mit Verlust der Prägung Stufe III ist und die Expression von nicht mehr als einem der geprägten Gene Z1, Z4, Z5, Z6, Z8, Z10, Z11, Z13 und Z16 mit Abweichung der Kopienzahl Stufe III ist; der kolorektale Tumor als ein moderater kolorektaler Krebs bewertet wird, wenn die Expression von mindestens zwei der geprägten Gene Z1, Z4, Z5, Z6, Z8, Z10, Z11, Z13 und Z16 mit Verlust der Prägung Stufe III ist, wenn die Expression von mindestens zwei der geprägten Gene Z1, Z4, Z5, Z6, Z8, Z10, Z11, Z13 und Z16 mit Abweichung der Kopienzahl Stufe III ist, oder wenn die Expression von nicht mehr als einem der geprägten Gene Z1, Z4, Z5, Z6, Z8, Z10, Z11, Z13 und Z16 mit Verlust der Prägung Stufe IV ist und die Expression von nicht mehr als einem der geprägten Gene Z1, Z4, Z5, Z6, Z8, Z10, Z11, Z13 und Z16 mit Abweichung der Kopienzahl Stufe IV ist; oder der kolorektale Tumor als ein fortgeschrittener kolorektaler Krebs bewertet wird, wenn die Expression von mindestens zwei der geprägten Gene Z1, Z4, Z5, Z6, Z8, Z10, Z11, Z13 und Z16 mit Verlust der Prägung Stufe IV ist, wenn die Expression von mindestens zwei der geprägten Gene Z1, Z4, Z5, Z6, Z8, Z10, Z11, Z13 und Z16 mit Abweichung der Kopienzahl Stufe IV ist.

2. Die Vorrichtung nach Anspruch 1, wobei die Kalkulation der Expression des geprägten Gens die Kalkulation der Expression eines von Z1, Z5, Z10 oder Z11, vorzugsweise Z1 oder Z11, noch bevorzugter Z11 umfasst.

3. Die Vorrichtung nach Anspruch 1, wobei die Kalkulation der Expression des geprägten Gens die Kalkulation der Expression einer Kombination von zwei geprägten Genen von Z1, Z5, Z10 oder Z11, vorzugsweise eine Kombination von Z1 und Z11 oder eine Kombination von Z1 und Z5 umfasst.

4. Die Vorrichtung nach Anspruch 1, wobei die Kalkulation der Expression des geprägten Gens die Kalkulation der Expression einer Kombination von geprägten Genen umfasst, wobei die Kombination eine Kombination von neun geprägten Genen Z1, Z4, Z5, Z6, Z8, Z10, Z11, Z13 und Z16 ist.

5. Die Vorrichtung nach Anspruch 1, wobei die Testprobe von Schritt (1) von menschlichem Gewebe und/oder Zellen abgeleitet ist.

6. Die Vorrichtung nach Anspruch 5, wobei die Testprobe ein Gewebe-Paraffin-Schnitt, eine Enteroskopie-Biopsie-Probe und/oder abgeblätterte Zellen im Stuhl ist.

7. Die Vorrichtung nach Anspruch 1, wobei die *in situ* Hybridisierung RNAscope *in situ* Hybridisierung ist.

8. Die Vorrichtung nach Anspruch 7, wobei die RNAscope *in situ* Hybridisierung unter Verwendung eines singleplex oder multiplex Farb-Assay-Kits oder eines singleplex oder multiplex Fluoreszenz-Assay-Kits, vorzugsweise eines singleplex rot/braun Farb-Assay-Kits oder multiplex Fluoreszenz-Assay-Kits durchgeführt wird.

9. Verwendung der Vorrichtung nach Anspruch 1 zur Detektion eines kolorektalen Tumors.

**Revendications**

1. Dispositif pour déterminer la bénignité ou la malignité d'une tumeur colorectale, comprenant les unités suivantes :

(1) une unité d'échantillonnage pour obtenir un échantillon à tester ;
(2) une unité de conception de sonde pour concevoir une sonde spécifique de la séquence d'un gène soumis à empreinte, dans lequel la sonde est une séquence à l'intérieur d'un intron du gène soumis à empreinte ;
(3) une unité de détection pour effectuer l'hybridation *in situ* de la sonde de l'étape (2) à l'échantillon à tester ;

(4) une unité d'analyse pour analyser des images microscopiques et déterminer le niveau d'expression du gène soumis à empreinte ;

dans lequel, l'unité d'analyse est exploitée en calculant l'expression du gène soumis à empreinte avec perte d'empreinte, l'expression du gène soumis à empreinte avec variation du nombre de copies et l'expression totale du gène soumis à empreinte, et en classant l'expression du gène soumis à empreinte avec perte d'empreinte et l'expression du gène soumis à empreinte avec variation du nombre de copies, pour classer l'expression du gène soumis à empreinte ;

dans lequel le gène soumis à empreinte est l'un quelconque ou plusieurs parmi Z1, Z4, Z5, Z6, Z8, Z10, Z11, Z13 ou Z16, et dans lequel le gène soumis à empreinte Z1 est Gnas, le gène soumis à empreinte Z5 est Mest, le gène soumis à empreinte Z10 est Gatm, et le gène soumis à empreinte Z11 est Grb10, le gène soumis à empreinte Z4 est Igf2r, le gène soumis à empreinte Z6 est Plagl1, le gène soumis à empreinte Z8 est Dcn, le gène soumis à empreinte Z13 est Sgce, et le gène soumis à empreinte Z16 est Snrpn/Snurf ;

dans lequel l'expression totale d'un gène soumis à empreinte, l'expression d'un gène soumis à empreinte avec perte d'empreinte et l'expression d'un gène soumis à empreinte avec variation du nombre de copies sont calculées par les formules suivantes :

$$\text{Expression totale d'un gène soumis à empreinte} = (b + c + d)/(a + b + c + d) \times 100\ \% \ ;$$

$$\text{Expression d'un gène soumis à empreinte normal} = b/(b + c + d) \times 100\ \% \ ;$$

$$\text{Expression d'un gène soumis à empreinte avec perte d'empreinte} = c/(b + c + d) \times 100\ \% \ ;$$

$$\text{Expression d'un gène soumis à empreinte avec variation du nombre de copies} = d/(b + c + d) \times 100\ \% \ ;$$

dans lequel,

$a$ représente le fait qu'après avoir effectué une coloration à l'hématoxyline sur une cellule, il n'y a pas de marque dans le noyau de la cellule, le gène soumis à empreinte n'a pas d'expression ;

$b$ représente le fait qu'après avoir effectué une coloration à l'hématoxyline sur une cellule, il y a une marque rouge/marron dans le noyau de la cellule, le gène soumis à empreinte existe ;

$c$ représente le fait qu'après avoir effectué une coloration à l'hématoxyline sur une cellule, il y a deux marques rouge/marron dans le noyau de la cellule, le gène soumis à empreinte perd l'empreinte ; et

$d$ représente le fait qu'après avoir effectué une coloration à l'hématoxyline sur une cellule, il y a plus de deux marqueurs rouge/marron dans le noyau de la cellule, le gène soumis à empreinte présente une variation du nombre de copies ;

dans lequel l'expression de Z1, Z11 ou Z16 avec perte d'empreinte, l'expression de Z1, Z11 ou Z16 avec variation du nombre de copies, et l'expression totale de Z1, Z11 ou Z16 sont classées en 5 classes :

Classe 0 : un quelconque ou plusieurs parmi : l'expression de Z1, Z11 ou Z16 avec perte d'empreinte inférieure à 15 %, l'expression de Z1, Z11 ou Z16 avec variation du nombre de copies inférieure à 0,8 %, ou l'expression totale de Z1, Z11 ou Z16 inférieure à 20 % ;

Classe I : un quelconque ou plusieurs parmi : l'expression de Z1, Z11 ou Z16 avec perte d'empreinte de 15 à 20 %, l'expression de Z1, Z11 ou Z16 avec variation du nombre de copies de 0,8 à 2 %, ou l'expression totale de Z1, Z11 ou Z16 de 20 à 30 % ;

Classe II : un quelconque ou plusieurs parmi : l'expression de Z1, Z11 ou Z16 avec perte d'empreinte de 20 à 25 %, l'expression de Z1, Z11 ou Z16 avec variation du nombre de copies de 2 à 3,5 %, ou l'expression totale de Z1, Z11 ou Z16 de 30 à 40 % ;

Classe III : un quelconque ou plusieurs parmi : l'expression de Z1, Z11 ou Z16 avec perte d'empreinte de 25 à 30 %, l'expression de Z1, Z11 ou Z16 avec variation du nombre de copies de 3,5 à 5 %, ou l'expression totale de Z1, Z11 ou Z16 de 40 à 50 % ; et

Classe IV : un quelconque ou plusieurs parmi : l'expression de Z1, Z11 ou Z16 avec perte d'empreinte de plus de 30 %, l'expression de Z1, Z11 ou Z16 avec variation du nombre de copies de plus de 5 %, ou l'expression totale de Z1, Z11 ou Z16 de plus de 50 % ; et

dans lequel l'expression de Z4, Z5, Z6, Z8, Z10 ou Z13 avec perte d'empreinte, l'expression de Z4, Z5, Z6, Z8, Z10 ou Z13 avec variation du nombre de copies, et l'expression totale de Z4, Z5, Z6, Z8, Z10 ou Z13 sont classées en 5 classes :

Classe 0 : un quelconque ou plusieurs parmi : l'expression de Z4, Z5, Z6, Z8, Z10 ou Z13 avec perte d'empreinte inférieure à 10 %, l'expression de Z4, Z5, Z6, Z8, Z10 ou Z13 avec variation du nombre de copies inférieure à 0,8 %, ou l'expression totale de Z4, Z5, Z6, Z8, Z10 ou Z13 inférieure à 15 % ;

Classe I : un quelconque ou plusieurs parmi : l'expression de Z4, Z5, Z6, Z8, Z10 ou Z13 avec perte d'empreinte de 10 à 20 %, l'expression de Z4, Z5, Z6, Z8, Z10 ou Z13 avec variation du nombre de copies de 0,8 à 1,5 %, ou l'expression totale de Z4, Z5, Z6, Z8, Z10 ou Z13 de 15 à 20 % ;

Classe II : un quelconque ou plusieurs parmi : l'expression de Z4, Z5, Z6, Z8, Z10 ou Z13 avec perte d'empreinte de 20 à 25 %, l'expression de Z4, Z5, Z6, Z8, Z10 ou Z13 avec variation du nombre de copies de 1,5 à 3 %, ou l'expression totale de Z4, Z5, Z6, Z8, Z10 ou Z13 de 20 à 30 % ;

Classe III : un quelconque ou plusieurs parmi : l'expression de Z4, Z5, Z6, Z8, Z10 ou Z13 avec perte d'empreinte de 25 à 30 %, l'expression de Z4, Z5, Z6, Z8, Z10 ou Z13 avec variation du nombre de copies de 3 à 5 %, ou l'expression totale de Z4, Z5, Z6, Z8, Z10 ou Z13 de 30 à 40 % ; et

Classe IV : un quelconque ou plusieurs parmi : l'expression de Z4, Z5, Z6, Z8, Z10 ou Z13 avec perte d'empreinte de plus de 30 %, l'expression de Z4, Z5, Z6, Z8, Z10 ou Z13 avec variation du nombre de copies de plus de 5 %, ou l'expression totale de Z4, Z5, Z6, Z8, Z10 ou Z13 de plus de 40 % ; et

dans lequel :

la tumeur colorectale est qualifiée de tumeur bénigne, si l'expression des gènes soumis à empreinte Z1, Z4, Z5, Z6, Z8, Z10, Z11, Z13 et Z16 avec perte d'empreinte et l'expression des gènes soumis à empreinte Z1, Z4, Z5, Z6, Z8, Z10, Z11, Z13 et Z16 avec variation du nombre de copies sont toutes deux inférieures à la classe I, ou si l'expression d'au plus un des gènes soumis à empreinte Z1, Z4, Z5, Z6, Z8, Z10, Z11, Z13 et Z16 avec perte d'empreinte sont de classe I et l'expression d'au plus un des gènes soumis à empreinte Z1, Z4, Z5, Z6, Z8, Z10, Z11, Z13 et Z16 avec variation du nombre de copies est de classe I ;

la tumeur colorectale est qualifiée de cancer colorectal potentiel, si l'expression d'au moins deux des gènes soumis à empreinte Z1, Z4, Z5, Z6, Z8, Z10, Z11, Z13 et Z16 avec perte d'empreinte est de classe I, si l'expression d'au moins deux des gènes soumis à empreinte Z1, Z4, Z5, Z6, Z8, Z10, Z11, Z13 et Z16 avec variation du nombre de copies est de classe I, ou si l'expression d'au plus un des gènes soumis à empreinte Z1, Z4, Z5, Z6, Z8, Z10, Z11, Z13 et Z16 avec perte d'empreinte est de classe II et l'expression d'au plus un des gènes soumis à empreinte Z1, Z4, Z5, Z6, Z8, Z10, Z11, Z13 et Z16 avec variation du nombre de copies est de classe II ;

la tumeur colorectale est qualifiée de cancer colorectal précoce, si l'expression d'au moins deux des gènes soumis à empreinte Z1, Z4, Z5, Z6, Z8, Z10, Z11, Z13 et Z16 avec perte d'empreinte est de classe II, si l'expression d'au moins deux des gènes soumis à empreinte Z1, Z4, Z5, Z6, Z8, Z10, Z11, Z13 et Z16 avec variation du nombre de copies est de classe II, ou si l'expression d'au plus un des gènes soumis à empreinte Z1, Z4, Z5, Z6, Z8, Z10, Z11, Z13 et Z16 avec perte d'empreinte sont de classe III et l'expression d'au plus un des gènes soumis à empreinte Z1, Z4, Z5, Z6, Z8, Z10, Z11, Z13 et Z16 avec variation du nombre de copies est de classe III ;

la tumeur colorectale est qualifiée de cancer colorectal modéré, si l'expression d'au moins deux des gènes soumis à empreinte Z1, Z4, Z5, Z6, Z8, Z10, Z11, Z13 et Z16 avec perte d'empreinte est de classe III, si l'expression d'au moins deux des gènes soumis à empreinte Z1, Z4, Z5, Z6, Z8, Z10, Z11, Z13 et Z16 avec variation du nombre de copies est de classe III, ou si l'expression d'au plus un des gènes soumis à empreinte Z1, Z4, Z5, Z6, Z8, Z10, Z11, Z13 et Z16 avec perte d'empreinte est de classe IV et l'expression d'au plus un des gènes soumis à empreinte Z1, Z4, Z5, Z6, Z8, Z10, Z11, Z13 et Z16 avec variation du nombre de copies est de classe IV ; ou

la tumeur colorectale est qualifiée de cancer colorectal avancé, si l'expression d'au moins deux des gènes soumis à empreinte Z1, Z4, Z5, Z6, Z8, Z10, Z11, Z13 et Z16 avec perte d'empreinte est de classe IV, ou si l'expression d'au moins deux des gènes soumis à empreinte Z1, Z4, Z5, Z6, Z8, Z10, Z11, Z13 et Z16 avec variation du nombre de copies est de classe IV.

2. Dispositif selon la revendication 1, dans lequel le calcul de l'expression du gène soumis à empreinte comprend le calcul de l'expression de l'un quelconque parmi Z1, Z5, Z10 ou Z11, de préférence Z1 ou Z11, et de manière davantage préférée Z11.

3. Dispositif selon la revendication 1, dans lequel le calcul de l'expression du gène soumis à empreinte comprend le calcul de l'expression d'une combinaison de deux gènes soumis à empreinte quelconques parmi Z1, Z5, Z10 ou Z11, de préférence une combinaison de Z1 et Z11 ou une combinaison de Z1 et Z5.

4. Dispositif selon la revendication 1, dans lequel le calcul de l'expression du gène soumis à empreinte comprend le calcul de l'expression d'une combinaison de de gènes soumis à empreinte, dans lequel la combinaison est une combinaison de neuf gènes soumis à empreinte Z1, Z4, Z5, Z6, Z8, Z10, Z11, Z13 et Z16.

5. Dispositif selon la revendication 1, dans lequel l'échantillon à tester de l'étape (1) est dérivé de tissus et/ou cellules humains.

6. Dispositif selon la revendication 5, dans lequel l'échantillon à tester est une section de tissu dans la paraffine, un échantillon de biopsie d'entéroscopie et/ou des cellules exfoliées dans les selles.

7. Dispositif selon la revendication 1, dans lequel l'hybridation *in situ* est une hybridation *in situ* RNAscope.

8. Dispositif selon la revendication 7, dans lequel l'hybridation *in situ* RNAscope est effectuée en utilisant un kit d'essai coloré uniplex ou multiplex ou un kit d'essai par fluorescence uniplex ou multiplex, de préférence un kit d'essai coloré rouge/marron uniplex ou un kit d'essai par fluorescence multiplex.

9. Utilisation du dispositif selon la revendication 1 pour la détection d'une tumeur colorectale.

Figure 1

Grade 0

Figure 2(a)

Grade I

Figure 2(b)

Grade II

Figure 2(c)

Grade III

Figure 2(d)

Grade IV

Figure 2(e)

Figure 3a

Figure 3b

Figure 3c

Figure 3d

Figure 3e

Figure 3f

## Z1

Figure 4a

## Z5

Figure 4b

## Z10

Figure 4c

Figure 4d

Figure 4e

Figure 4f

Figure 4g

Figure 4h

Figure 4i

Figure 5a

Figure 5b

Figure 5c

Figure 5d

Figure 5e

Figure 5f

Z8

Figure 5g

Z13

Figure 5h

Z16

Figure 5i

Normal

Figure 6(a)

Malignant

Figure 6(b)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZHONG et al.** *Scientific Reports,* 2016, vol. 6 (1